(19) Europäisches Patentamt
European Patent Office
Office européen des brevets

(11) Veröffentlichungsnummer: **0 402 751 B1**

(12) **EUROPÄISCHE PATENTSCHRIFT**

(45) Veröffentlichungstag der Patentschrift: **04.05.94**

(21) Anmeldenummer: **90110674.0**

(22) Anmeldetag: **06.06.90**

Die Akte enthält technische Angaben, die nach dem Eingang der Anmeldung eingereicht wurden und die nicht in dieser Patentschrift enthalten sind.

(51) Int. Cl.5: **C07D 239/60**, C07D 403/12, C07D 409/12, C07D 231/12, C07D 249/08, C07D 231/16, C07D 239/52, C07D 213/55, C07D 233/06, C07D 251/30, C07D 335/02

# (54) Salicylaldehyd- und Salicylsäurederivate sowie deren Schwefelanaloge, Verfahren zu ihrer Herstellung und ihre Verwendung als Herbizide und Bioregulatoren.

(30) Priorität: **14.06.89 DE 3919435**

(43) Veröffentlichungstag der Anmeldung:
**19.12.90 Patentblatt 90/51**

(45) Bekanntmachung des Hinweises auf die Patenterteilung:
**04.05.94 Patentblatt 94/18**

(84) Benannte Vertragsstaaten:
**AT BE CH DE ES FR GB IT LI NL**

(56) Entgegenhaltungen:
**EP-A- 0 223 406**
**EP-A- 0 242 081**
**EP-A- 0 287 072**
**FR-A- 1 451 179**

(73) Patentinhaber: **BASF Aktiengesellschaft**
**Carl-Bosch-Strasse 38**
**D-67063 Ludwigshafen(DE)**

(72) Erfinder: **Vogelbacher, Uwe Josef Dr.**
**Niedererdstrasse 56**
**D-6700 Ludwigshafen(DE)**
Erfinder: **Eicken, Karl, Dr.**
**Am Huettenwingert 12**
**D-6706 Wachenheim(DE)**
Erfinder: **Rheinheimer, Joachim, Dr.**
**Merziger Strasse 24**
**D-6700 Ludwigshafen(DE)**
Erfinder: **Goetz, Norbert, Dr.**
**Schoefferstrasse 25**
**D-6520 Worms 1(DE)**
Erfinder: **Harreus, Albrecht, Dr.**
**Teichgasse 13**
**D-6700 Ludwigshafen(DE)**

EP 0 402 751 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 111, Nr. 17, 23. Oktober 1989, Columbus, Ohio, USAKATO TSUGIHIRO et al. "2- -(2-Pyridyl)-pyrimidine derivatives processes fortheir preparation and plant fungicides containing them" Seite 728,Zusammenfassung- -Nr. 153 831g**

Erfinder: **Paul, Gerhard, Dr.**
**Berner Weg 34**
**D-6700 Ludwigshafen(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

## Beschreibung

Die vorliegende Erfindung betrifft Salicylaldehyd- und Salicylsäurederivate und deren Schwefelanaloge der Formel I,

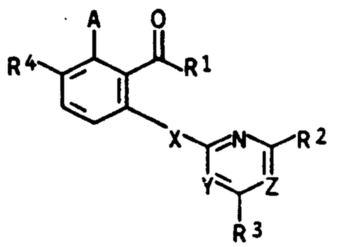

I

in der die Substituenten folgende Bedeutung haben:

$R^1$ Wasserstoff
eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
einen Rest -$OR^5$ oder einen Rest $ON=CR^6R^7$, worin
$R^5$
Wasserstoff, ein Alkalimetallkation, das Äquivalent eines Erdalkalimetallkations oder ein organisches Ammoniumion;
eine $C_3$-$C_{12}$-Cycloalkylgruppe, welche ein bis drei $C_1$-$C_4$-Alkylreste tragen kann;
eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, $C_3$-$C_{12}$-Cycloalkyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
eine $C_1$-$C_{10}$-Alkylgruppe, welche ein bis fünf Halogenatome tragen kann und einen der folgenden Reste trägt: ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;
eine $C_3$-$C_6$-Alkenyl- oder eine $C_3$-$C_6$-Alkinylgruppe, wobei diese Gruppen ihrerseits ein bis fünf Halogenatome tragen können;
unsubstituiertes oder ein- bis dreifach durch $C_1$-$C_4$-Alkyl oder $C_1$-$C_4$-Alkoxy substituiertes oder ein- bis fünffach durch Halogen substituiertes Phenyl;
bedeutet und
$R^6$ und $R^7$
$C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann, bedeuten;

$R^2$, $R^3$ $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

X ein Sauerstoff- oder Schwefelatom;

Y,Z ein Stickstoffatom oder eine Methingruppe =CH-;

$R^4$ Wasserstoff, ein Halogenatom, $C_1$-$C_4$-Alkyl, Cyano oder $C_1$-$C_4$-Halogenalkyl;

A einen gegebenenfalls ein bis dreifach substituierten, im Fall von Halogen als Substituent ein bis fünffach substituierten Phenylrest

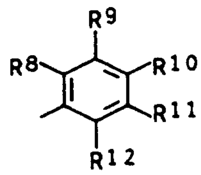

worin
$R^8$ - $R^{12}$ Wasserstoff, Halogen, Cyano, Nitro;
eine $C_3$-$C_6$-Alkenyl-, $C_3$-$C_6$-Alkenyloxy-, $C_3$-$C_6$-Alkinyloxy- oder eine $C_3$-$C_6$-Alkinylgruppe, wobei diese Gruppen ihrerseits ein bis fünf Halogenatome tragen können;
Di-$C_1$-$C_4$-alkylamino, $C_3$-$C_8$-Cycloalkyl, welches ein bis drei $C_1$-$C_4$-Alkylreste tragen kann;
$C_1$-$C_{10}$-Alkoxycarbonyl, $C_1$-$C_4$-Alkylthio;
eine Phenoxygruppe, wobei der aromatische Rest ein bis fünf Halogenatome und/oder ein bis drei der folgenden Reste tragen kann, $C_1$-$C_4$-Alkyl, $C_1$-$C_4$-Halogenalkyl, $C_1$-$C_4$-Halogenalkoxy, $C_1$-$C_4$-Alkoxy, $C_1$-$C_4$-Alkylthio;
eine $C_1$-$C_{10}$-Alkyl- oder Alkoxygruppe, welche ein bis fünf Halogenatome und/oder einen der folgenden Reste tragen kann: $C_1$-$C_4$-Alkoxy, $C_1$-$C_4$-Alkylthio, Phenyl oder Phenoxy, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Halogenalkoxy, $C_1$-$C_4$-Alkylthio;
bedeuten;
einen 5-gliedrigen Heteroaromaten, mit zwei bis vier Stickstoffatomen oder ein bis zwei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, $C_1$-$C_4$-Alkyl, $C_1$-$C_4$-Alkylthio, $C_1$-$C_4$-Halogenalkyl oder Phenyl, das unsubstituiert oder durch ein bis drei Halogenatome und/oder ein bis drei Methylgruppen substituiert ist;
einen Thienylrest, der ein bis drei Halogenatome und/oder einen bis drei der folgenden Reste tragen kann: $C_1$-$C_4$-Alkyl, $C_1$-$C_2$-Halogenalkyl oder Nitro;
einen Pyridylrest, der ein bis drei Halogenatome und/oder einen bis drei der folgenden Reste tragen kann: $C_1$-$C_4$-Alkyl, $C_1$-$C_2$-Halogenalkyl oder Nitro;
einen Naphthyl-, Chinolin-, Indazolyl- oder Benztriazolylrest, welcher jeweils ein bis drei Halogenatome und/oder einen bis drei der folgenden Reste tragen kann: $C_1$-$C_4$-Alkyl oder $C_1$-$C_2$-Halogenalkyl,
sowie umweltverträgliche Salze der Verbindungen I, beispielsweise Alkalimetallsalze, Erdalkalimetallsalze, Mangan-, Kupfer-, Zink- oder Eisensalze sowie Ammonium-, Phosphonium-, Sulfonium- oder Sulfoxoniumsalze.

Weiterhin betrifft die Erfindung Verfahren zur Herstellung der Verbindungen I sowie ihre Verwendung als Herbizide und Wachstumsregulatoren sowie neue Salicylsäurederivate der Formel II'

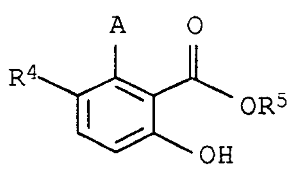

II'

als Zwischenprodukte zur Herstellung der Verbindungen I.

In der Formel II' haben die Reste $R^5$, $R^4$ und A die folgende Bedeutung:

$R^5$ Wasserstoff, ein Alkalimetallkation, das Äquivalent eines Erdalkalimetallkations, ein organisches Ammoniumion; eine $C_1$-$C_{10}$-Alkylgruppe, welche ein bis fünf Halogenatome und/oder einen der folgenden Reste tragen kann: $C_1$-$C_4$-Alkoxy, $C_1$-$C_4$-Alkylthio, Phenyl, Phenoxy, wobei die Phenylreste jeweils ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

$R^4$ Wasserstoff, ein Halogenatom, $C_1$-$C_4$-Alkyl, Cyano oder $C_1$-$C_4$-Halogenalkyl;

A einen 5-gliedrigen Heteroaromaten, mit zwei bis vier Stickstoffatomen oder ein bis zwei Stickstoffatomen sowie zusätzlich einem Schwefel- oder Sauerstoffatom im Ring, welcher ein bis drei

Halogenatome und/oder einen bis drei der folgenden Reste tragen kann: Nitro, Cyano, $C_1$-$C_4$-Alkyl, $C_1$-$C_4$-Alkylthio, $C_1$-$C_4$-Halogenalkyl oder Phenyl, das unsubstituiert oder durch ein bis drei Halogenatome und/oder ein bis drei Methylgruppen substituiert ist;

einen Thienylrest, der ein bis drei Halogenatome und/oder einen bis drei der folgenden Reste tragen kann: $C_1$-$C_4$-Alkyl, $C_1$-$C_2$-Halogenalkyl oder Nitro;

einen Naphthyl-, Chinolin-, Indazolyl- oder Benztriazolylrest, welcher jeweils ein bis drei Halogenatome und/oder einen bis drei der folgenden Reste tragen kann: $C_1$-$C_4$-Alkyl oder $C_1$-$C_2$-Halogenalkyl.

In der Literatur (EP-A 223 406, EP-A 249 708, EP-A 287 072 und EP-A 287 079) sind herbizid wirksame substituierte Salicylsäuren und deren Schwefelanaloge beschrieben. Ihre Wirkung ist jedoch unbefriedigend.

Dem Stand der Technik sind ferner 6-Phenylsalicylsäure, deren Methyl- und Ethylester (W. Staedel, Chem. Ber. 28 (1895) 111, G. Heyl, Chem. Ber. 31 (1898) 3022, G. Heyl, J. Prakt. Chem. 59 (1899) 434 sowie T.M. Harris et al., J. Chem. Soc. Chem. Com. (1974) 362-363) zu entnehmen. 6-(3-Pyridyl)-salicylsäure wird von Prelog et al. in Helv. Chim. Acta 30 (1948) 675-689 erwähnt.

Aufgabe der vorliegenden Erfindung waren daher neue Salicylsäurederivate bzw. deren Schwefelanaloge mit verbesserten herbiziden Eigenschaften sowie mit pflanzenwachstumsregulierenden Eigenschaften.

Entsprechend dieser Aufgabe wurden die eingangs definierten Verbindungen der Formel I gefunden. Außerdem wurden Verfahren zur Herstellung der Verbindungen I und Verfahren zur Bekämpfung unerwünschten Pflanzenwuchses mit den Verbindungen I gefunden. Es wurde außerdem gefunden, daß Salicylsäurederivate der vorstehend definierten allgemeinen Formel I ausgezeichnete pflanzenwachstumsregulierende Eigenschaften besitzen. Als Zwischenprodukte zur Herstellung der Verbindungen I wurden die neuen Salicylsäurederivate II' gefunden.

Verbindungen der Formel I erhält man beispielsweise, indem man ein entsprechend substituiertes Salicylsäurederivat der Formel II, das in Einzelfällen bekannt ist oder nach üblichen Methoden ausgehend von bekannten Vorprodukten hergestellt werden kann, mit einer entsprechenden Verbindung der Formel III in Gegenwart einer Base umsetzt.

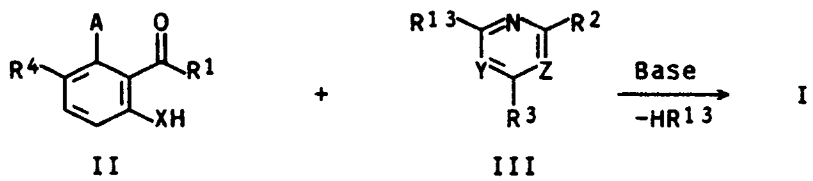


$R^{13}$ in Formel III bedeutet eine übliche nucleofuge Abgangsgruppe, beispielsweise Halogen wie Chlor, Brom und Iod, Aryl- oder Alkylsulfonyl wie Toluolsulfonyl und Methylsulfonyl oder eine andere äquivalente Abgangsgruppe. Verbindungen der Formel III mit einem reaktionsfähigen Substituenten $R^{13}$ sind bekannt oder mit dem allgemeinen Fachwissen leicht zu erhalten. Als Base können Alkali- oder Erdalkalimetallhydride wie NaH und $CaH_2$, Alkalimetallhydroxide wie NaOH und KOH, Alkalimetallalkohole wie Kalium-tert.-butylat, Alkalimetallcarbonate wie $Na_2CO_3$ und $K_2CO_3$, Alkalimetallamide wie $NaNH_2$ und Lithiumdiisopropylamid oder tertiäre Amine Verwendung finden. Bei Einsatz einer anorganischen Base kann man einen Phasentransferkatalysator zusetzen, wenn dies den Umsatz fördert.

Soweit es sich bei den in der beschriebenen Weise hergestellten Verbindungen der Formel I um Carbonsäuren handelt (wenn also $R^1$ Hydroxyl bedeutet), können hieraus andere beschriebene Verbindungen z.B. auch dadurch hergestellt werden, daß man die Carbonsäure zunächst auf übliche Weise in eine aktivierte Form wie ein Halogenid oder Imidazolid überführt und dieses dann mit der entsprechenden Hydroxylverbindung umsetzt. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Die Zwischenprodukte der Formel II lassen sich, wenn X für ein Sauerstoffatom und A für einen über ein Kohlenstoffatom gebundenen Aromaten bzw. Heteroaromaten steht, gemäß dem untenstehenden Schema aus einer 1,3-Dicarbonylverbindung IV (mit $R^5$ = gegebenenfalls phenylsubstituiertes $C_1$-$C_{10}$-Alkyl, insbesondere $C_1$-$C_4$-Alkyl) und einem $\alpha,\beta$-ungesättigten Keton V aufbauen:

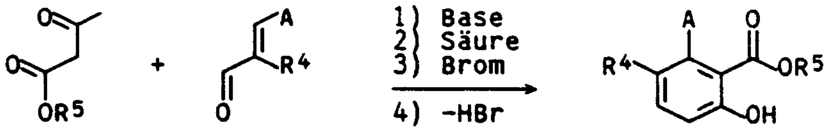


IV V II'

(A = über C-Atom gebundener Aromat oder Heteroaromat)

untenstehendem Schema aus einem Methylenphosphoran IV' und einem α,β-ungesättigten Keton V herstellen:

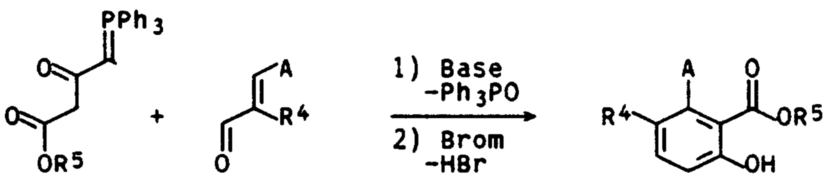


IV' V II'

(A = über C-Atom gebundener Aromat oder Heteroaromat)

Dabei sind die Verbindungen IV, IV' und V im allgemeinen bekannt oder nach den üblichen Verfahren leicht herstellbar. Als Base kommen die oben genannten Verbindungen in Frage. Als Säure eignen sich starke Säuren, beispielsweise Chlorwasserstoff, Bromwasserstoff, Tetrafluoroborsäure, Toluolsulfonsäure oder Trifluoressigsäure. Die Bromwasserstoffabspaltung kann thermisch oder in Gegenwart einer Base, beisielsweise eines organischen Amins, durchgeführt werden.

Steht A in Formel II für einen über ein Stickstoffatom gebundenen Heteroaromaten und X für ein Sauerstoffatom, so kann man dieses Zwischenprodukt gemäß dem folgenden Schema aufbauen:

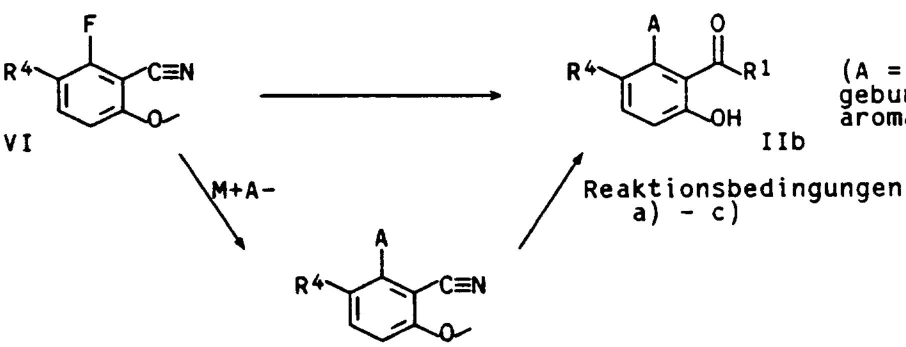


VI IIb (A = über N gegebundener Heteroaromat)

VII

a) 1. HCl/Ethanol
2. HCl/Wasser
3. $BBr_3$

b) 1. OH-/Wasser
2. $H^+$/Wasser
3. HBr, HI oder $BBr_3$

c) 1. $H^+$/Wasser
2. HBr, HI oder $BBr_3$

Dabei steht M + A- für das jeweilige Alkalimetallazolid. Als Alkohole kommen bei der Spaltung des Nitrils VII nach Variante a) insbesondere $C_1$-$C_4$-Alkylalkohole in Frage.

Die wie oben beschrieben hergestellten Zwischenprodukte der Formel II fallen üblicherweise als Alkylester an. Diese lassen sich nach den bekannten Verfahren zu den Carbonsäuren hydrolysieren. Man kann letztere nun nach literaturbekannten Verfahren in verschiedene Ester überführen, die man zur Darstellung von Wirkstoffen der Formel I gemäß Anspruch 1 benötigt.

Wahlweise kann man auch die Zwischenprodukte der Formel VII nach allgemein bekannten Methoden mit Alkali- oder Tetraalkylammonium-hydroxiden zu den entsprechenden Amiden und dann mit Mineralsäuren, z.B. konz. Salzsäure, zu den Carbonsäuren und anschließend mit konz. Bromwasserstoffsäure zu den

Salicylsäuren IIb umsetzen. Diese Schritte können bei Bedarf ohne Isolierung der Zwischenstufen durchgeführt werden.

Die Verbindungen der Formel I können auch in der Weise hergestellt werden, daß man die freie Salicylsäure bzw. ihre Schwefelanalogen der Formel I'

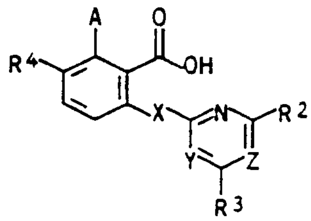

I'

zunächst in an sich bekannter Weise in das Halogenid oder eine andere aktivierte Form der Carbonsäure überführt und diese dann gegebenenfalls in Gegenwart einer anorganischen oder organischen Base mit einem gegebenenfalls substituierten Alkohol, einem Azol, einem Oxim oder N-Hydroxysuccinylimin umsetzt.

Im Hinblick auf die herbizide Wirksamkeit sind Verbindungen I bevorzugt, in denen die Substituenten folgende Bedeutung haben:

$R^1$ Wasserstoff, Succinyliminooxy,
5-gliedriges Heteroaryl wie Pyrrolyl, Pyrazolyl, Imidazolyl und Triazolyl, insbesondere Imidazolyl und Pyrazolyl, wobei der aromatische Rest über Stickstoff gebunden ist und seinerseits ein bis vier Halogenatome wie vorstehend genannt, insbesondere Fluor und Chlor und/oder ein bis zwei der folgenden Reste tragen kann:
Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl,
Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;
Alkoxy wie vorstehend genannt, mit ein bis vier Kohlenstoffatomen, Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und/oder
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
ein Rest $OR^5$, worin

$R^5$ Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion;
Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, welches ein bis fünf der vorstehend genannten Halogenatome, insbesondere Fluor und Chlor und/oder einen der folgenden Reste tragen kann:
Cyano, Alkoxy bzw. Alkylthio mit ein bis vier Kohlenstoffatomen wie vorstehend genannt, insbesondere Methoxy, Ethoxy, 1-Methylethoxy und Methylthio;
Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;

Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere Methoxycarbonyl, Ethoxycarbonyl, 1-Methylethoxycarbonyl und 1-Methylpropyloxycarbonyl.

Phenyl, Phenoxy oder Phenylcarbonyl, wobei diese aromatischen Reste ihrerseits ein bis fünf Halogenatome wie vorstehend genannt, insbesondere Fluor, Chlor und Brom und/oder ein bis drei der folgenden Reste tragen können:

Alkyl, Halogenalkyl, Alkoxy, Halogenalkoxy und/oder Alkylthio mit jeweils ein bis vier Kohlenstoffatomen wie im allgemeinen und im besonderen vorstehend genannt, oder

$C_1$-$C_{10}$-Alkyl wie vorstehend genannt, welches ein bis fünf Halogenatome wie Fluor, Chlor, Brom oder Iod, insbesondere Fluor oder Chlor tragen kann und zusätzlich einen der folgenden Reste trägt:

5-gliedriges Heteroaryl mit ein bis drei Stickstoffatomen wie vorstehend für $R^1$ genannt.

$C_2$-$C_6$-Alkyl, insbesondere $C_2$-$C_4$-Alkyl, das in 2-Stellung substituiert ist durch $C_1$-$C_6$-Alkoxyimino, z.B. Methoxy-, Ethoxy- oder Propoxyimino; $C_3$-$C_6$-Alkenyloxyimino wie 2-Propenyloxyimino, 2-Butenyloxyimino, 3-Butenyloxyimino; $C_3$-$C_6$-Halogenalkenyloxyimino wie 3,3-Dichlor-2-propenyloxyimino, 2,3,3-Trichlor-2-propenoxyimino oder Benzyloxyimino.

Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;

Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl, wobei diese Alkenyl- und Alkinylgruppen ein bis fünf der vorstehend im allgemeinen und im besonderen genannten Halogenatome tragen können, bedeutet;

$C_3$-$C_{12}$-Cycloalkyl, insbesondere $C_3$-$C_6$-Cycloalkyl wie Cyclopropyl, Cyclobutyl, Cyclopentyl, Cyclohexyl, das gegebenenfalls durch ein bis drei $C_1$-$C_4$-Alkylreste substituiert ist;

Phenyl, Phenyl ein bis dreifach substituiert durch $C_1$-$C_4$-Alkyl oder- Alkoxy wie Methyl, Ethyl, Propyl, Butyl, Methoxy, Ethoxy oder Phenyl substituiert durch ein bis fünf Halogenatome, z.B. Chlor oder Fluor;

ein Rest $ON=CR^6R^7$, worin

$R^6$ $R^7$ unverzweigtes oder verzweigtes $C_1$-$C_{20}$-Alkyl, vorzugsweise $C_1$-$C_{15}$-Alkyl, insbesondere $C_1$-$C_9$-Alkyl, welches einen Phenyl, einen $C_1$-$C_4$-Alkoxy oder einen $C_1$-$C_4$-Alkylthiorest tragen

kann, Phenyl oder gemeinsam $C_3$-$C_{12}$-Alkylen, vorzugsweise $C_4$-$C_7$-Alkylen, welches ein bis drei $C_1$-$C_3$-Alkylgruppen, vorzugsweise Methyl- oder Ethylgruppen tragen kann, bedeutet;

$R^2$, $R^3$ im allgemeinen und im besonderen die bei $R^1$ genannten Alkylgruppen, Halogenalkylgruppen, Alkoxygruppen, Halogenalkoxygruppen und/oder Alkylthiogruppen mit jeweils ein bis vier Kohlenstoffatomen;

X Sauerstoff oder Schwefel;

Y, Z Stickstoff oder eine Methingruppe =CH-;

$R^4$ Wasserstoff; Halogen wie bei $R^1$ genannt, insbesondere Fluor, Chlor und Brom;
Cyano;
Alkyl mit jeweils ein bis vier Kohlenstoffatomen, insbesondere ein bis drei Kohlenstoffatomen welches ein- bis fünffach durch Halogen, insbesondere Fluor und Chlor substituiert ist. Beispielsweise seien Methyl, Ethyl, n-Propyl, iso-Propyl, Trichlormethyl und Trifluormethyl genannt.

A unsubstituiertes oder substituiertes Phenyl, worin als Substituenten $R^8$ bis $R^{12}$ in Betracht kommen: Halogen wie Fluor, Chlor, Brom oder Iod; Cyano; Nitro, ggf. halogensubstituiertes Alkenyl, Alkenyloxy, Alkinyloxy oder Alkinyl mit jeweils 3 bis 6 Kohlenstoffatomen; Di-$C_1$-$C_4$-alkylamino wie Di-Methylamino, Di-Ethylamino, Di-Propylamino, Di-1-Methylethylamino, Di-Butylamino, Di-1-Methylpropylamino, Di-2-Methylpropylamino, Di-1,1-Dimethylethylamino, Ethylmethylamino, Propylmethylamino, 1-Methylethylmethylamino oder Butylmethylamino; gegebenenfalls alkylsubstituiertes Cycloalkyl wie vorstehend für $R^5$ aufgeführt, Alkoxycarbonyl oder Alkylthio wie vorstehend für $R^5$ aufgeführt, gegebenenfalls substituiertes Phenoxy wie unter $R^5$ aufgeführt, $C_1$-$C_{10}$-Alkyl- oder Alkoxy, insbesondere $C_1$-$C_6$, bevorzugt $C_1$-$C_4$-Alkyl oder Alkoxy welche unsubstituiert oder durch die genannten Reste substituiert sind; beispielsweise seien die folgenden substituierten Phenylreste für A genannt:
2-Fluorphenyl, 3-Fluorphenyl, 4-Fluorphenyl, 2,6-Difluorphenyl, 2,4-Diflurophenyl, 2-Fluor-4-trifluormethylphenyl, 2,3-Difluorphenyl, 2-Chlorphenyl, 3-Chlorphenyl, 2-Iodphenyl, 2-Bromphenyl, 2-Chlor-6-Fluorphenyl, Pentafluorphenyl, Pentachlorphenyl, 2,4-Dichlorphenyl, 2,6-Dichlorphenyl, 2-Chlor-4-fluorphenyl, 3,5-Dichlorphenyl, 2-Chlor-6-methylphenyl, 2,3,5-Trichlorphenyl, 2-Methylphenyl, 3-Methylphenyl, 4-Methylphenyl, 2,6-Dimethylphenyl, 2,4-Dimethylphenyl, 3,5-Dimethylphenyl, 2,4,6-Trimethylphenyl, 2-Chlor-4-methylphenyl, 2-Methoxyphenyl, 4-Methoxyphenyl, 4-Chlor-2-methoxyphenyl, 2-Trifluormethylphenyl, 2,3-Dimethyl-4-methoxyphenyl, 4-Dimethylamino-2-methylphenyl, 3-Cyanophenyl, 3-Nitrophenyl, 3-Phenoxyphenyl, 3-(3-Trifluormethylphenoxy)phenyl, 3-Trifluormethylphenyl,
gegebenenfalls substituiertes 5-gliedriges Hetaryl mit 2 bis 4 Stickstoffatomen wie für $R^1$ genannt oder ein bis 2 Stickstoffatomen und zusätzlich einem Schwefel- oder Sauerstoffatom wie gegebenenfalls substituiertes 5-gliedriges Hetaryl mit 2 bis 4 Stickstoffatomen wie für $R^1$ genannt oder ein bis 2 Stickstoffatomen und zusätzlich einem Schwefel- oder Sauerstoffatom wie Isoxazolyl, Oxazolyl, Thiazolyl, Thiadiazolyl.
Beispielsweise seien folgende Hetarylreste genannt:
Pyrazol-1-yl, 4-Methylpyrazol-1-yl, 3,5-Dimethylpyrazol-1-yl, 3,4,5-Trimethylpyrazol-1-yl, 4-Chlorpyrazol-1-yl, 4-Phenylpyrazol-1-yl, 4-Isopropylpyrazol-1-yl, 4-Nitropyrazol-1-yl,Imidazol-1-yl, 4,5-Dimethylimidazolyl, 2-Methyl-4,5-dichlorimidazolyl, 4(5)-Nitroimidazol-1-yl, [1,2,4]-Triazol-1-yl, 3(5)-Methyl-[1,2,4]-triazol-1-yl, [1,2,3]-Triazol-1-yl, 4,5-Dimethyl-[1,2,3]-triazol-1-yl, [1,2,3,4]-Tetrazol-1-yl, 1-Methylpyrazol-4-yl, 1-Phenylpyrazol-4-yl, 1,3,5-Trimethylpyrazol-4-yl, 1-Methylpyrazol-5-yl, 1-Phenylpyrazol-5-yl, 1-Methylpyrazol-3-yl, 1-Phenylpyrazol-3-yl, 1-Methylimidazol-2-yl, 1-Methylimidazol-5-yl, 1-Phenylimidazol-5-yl, 1-Phenyl-[1,2,3]-triazol-4-yl, Isoxazol-5-yl, Isoxazol-4-yl, 3-Methyl-isoxazol-5-yl, 3-Isopropyl-isoxazol-5-yl, 3-Phenyl-isoxazol-5-yl, Oxazol-2-yl, 2-Methyloxazol-4-yl, Thiazol-4-yl, 2-Benzthiazol-4-yl, 4-Methylthiazol-2-yl, 4-Methylthioazol-5-yl, 4-Phenylthiazol-2-yl, 2-Phenylthiazol-5-yl.
Gegebenenfalls substituierte Thienyl-, Pyridyl-, Naphthyl-, Chinolin-, Indazolyl- oder Benztriazolylreste für A sind beispielsweise 2-Thienyl, 3-Thienyl, 2,3-Dichlor-4-thienyl, 2-Methyl-5-thienyl, 2-Nitro-5-thienyl, 2-Pyridyl, 3-Pyridyl, 4-Pyridyl, 6-Methyl-2-pyridyl, 1-Naphthyl, 2-Napthyl, Chinolin-2-yl, Chinolin-4-yl, Chinolin-8-yl, 7-Chlor-chinolin-8-yl, 1-Indazolyl, 1-Benztriazolyl.

Insbesondere bevorzugt sind Verbindungen der Formel I, in denen

$R^2$ Methoxi, Methyl, Difluormethoxi oder Chlor;

$R^3$ Methoxi, Methyl, Difluormethoxi oder Chlor;

$R^4$ Wasserstoff oder Methyl;

bedeuten und, in denen X Sauerstoff, Y Stickstoff und Z eine Methingruppe darstellen und $R^1$ und A die im Anspruch genannte Bedeutung haben.

Ferner sind besonders bevorzugt Salicylaldehyd- und Salicylsäurederivate der Formel I, in der $R^2$ $OCH_3$, X Sauerstoff, Y Stickstoff, Z die Methingruppe und A sowie die Reste $R^1$, $R^3$ und $R^4$ die in Anspruch 1 genannte Bedeutung haben.

Als Salze der Verbindungen der Formel I kommen umweltverträgliche Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.004 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.005 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und

feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.024 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.063 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.003 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 2.004 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.004 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.024 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 3,0, vorzugsweise 0,005 bis 0,5 kg/ha aktive Substanz (a.S.).

Die wachstumsregulierend wirkenden Salicylsäurederivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel Ia im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden

kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Salicylsäurederivate der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wirkstoffe der Formel I können den Kulturpflanzen sowohl vom Samen her (als Saatgutbeizmittel) als auch über den Boden, d.h. durch die Wurzel, sowie - besonders bevorzugt - durch Spritzung über das Blatt zugeführt werden.

Infolge der hohen Plfanzenverträglichkeit kann die Aufwandmenge stark variiert werden.

Bei der Saatgutbehandlung werden im allgemeinen Wirkstoffmengen von 0,001 bis 50 g, vorzugsweise 0,01 bis 10 g, je Kilogramm Saatgut, benötigt.

Für die Blatt- und Bodenbehandlung sind im allgemeinen Gaben von 0,001 bis 10 kg/ha, bevorzugt 0,01 bis 3 kg/ha, insbesondere 0,01 bis 0,5 kg/ha als ausreichend zu betrachten.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |

| Botanischer Name | Deutscher Name |
|---|---|
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt. Verbindungen ohne diese Angaben lassen sich aus den entsprechenden Edukten analog synthetisieren. Die in der Tabelle wiedergegebenen Strukturen beschreiben besonders bevorzugte Wirkstoffe der Formel I.

Beispiel 1

3-(4,6-Dimethoxypyrimidin-2-yl)oxy-biphenyl-2-carbonsäuremethylester (Verbindung Nr. 1005):

a) 6-Phenylsalicylsäuremethylester:
Variante 1
58 g (0,5 mol) Acetessigsäuremethylester gibt man zu einer Lösung von 0,5 g Natriumhydrid in 100 ml getrocknetem Ethanol, tropft 66 g (0,5 mol) Zimtaldehyd schnell zu und rührt ca. 10 h bei Raumtemperatur. Die Mischung wird dann bei 0 °C mit trockenem HCl-Glas gesättigt und anschließend ca. 1 d bei Raumtemperatur (20 °C) nachgerührt. Man entfernt das Lösungsmittel und destilliert den verbliebenen Rückstand im Vakuum, wobei zunächst HCl-Glas freigesetzt wird. Die von 115 bis 165 °C/0,2 mbar übergehenden Fraktionen werden vereinigt und an Kieselgel (Eluation mit Toluol/Cyclohexan) chromatographiert. Man erhält 33,7 g eines gelben Öls als Zwischenprodukt. Man löst dieses in 150 ml Methylenchlorid und versetzt bei 0 °C mit einer Lösung von 22,9 g (0,14 mol) Brom in 150 ml Eisessig zügig, wärmt langsam auf und kocht eine Stunde unter Rückfluß. Die Reaktionslösung wird zu 200 ml Methylenchlorid und 500 ml Wasser gegossen. Die organische Phase wird abgetrennt und in üblicher Weise aufgearbeitet. Man erhält einen gelben Feststoff als Zwischenprodukt, der zur Weiterverarbeitung in 400 ml Methyl-t.-butylether gelöst wird. Man versetzt mit 55 g (0,56 mol) Triethylamin und kocht 5 h unter Rückfluß. Anschließend fügt man 300 ml Wasser zu, trennt die organische Phase ab und arbeitet in üblicher Weise auf. Der Rückstand wird bei 100 bis 114 °C/0,2 mbar destilliert. Ausbeute: 11,9 g.
Variante 2
Zur Lösung von 57 g (0,15 mol) 4-(Triphenylphosphoranyliden)-acetessigsäuremethylester in 700 ml

THF gibt man portionsweise unter Stickstoffatmosphäre 8,55 g (0,3 mol) Natriumhydrid (85 %) und erwärmt danach auf ca. 35 °C. Bei dieser Temperatur gibt man nun portionsweise 20 g (0,15 mol) Zimtaldehyd und anschließend 5 - 10 Tropfen Wasser hinzu, wobei die Reaktion anfangs exotherm verläuft und gekühlt werden muß. Es wird nun solange bei 35 °C weitergeführt, bis das Ylid vollständig umgesetzt ist (DC-Kontrolle, ca. 12-14 h). Die Reaktionsmischung wird nun mit 10 %iger Salzsäure sauer gestellt und nach dem Zufügen von 1 l Wasser viermal mit je 150 ml Ether extrahiert. Die vereinten Etherphasen werden mit 200 ml Wasser und 200 ml gesättigter Kochsalzlösung ausgeschüttelt und nach dem Trocknen über Natriumsulfat eingeengt. Der zurückbleibende schwarzbraune Rückstand wird mit 400 ml Methyl-tert.-butylether aufgenommen und mehrere Stunden gekocht. Nach dem Abkühlen filtriert man vom unlöslichen Rückstand (Triphenylphosphinoxid) ab und engt das Filtrat ein. Das zurückbleibende Öl wird durch Chromatographie an Kieselgel gereinigt (Laufmittel: Toluol/Essigester mit nach und nach ansteigendem Essigester-Anteil). Alle Fraktionen vor dem Triphenylphosphinoxid werden vereint und analog obiger Vorschrift bromiert, aromatisiert und aufgearbeitet. Man erhält 14,5 g des Produktes.

b) 3-(4,6-Dimethoxypyrimidin-2-yl)oxy-biphenyl-2-carbonsäuremethylester:

11,4 g (0,05 mol) 3-Hydroxy-biphenyl-2-carbonsäure-methylester werden in 50 ml getrocknetem Dimethylformamid vorgelegt und bei 0 bis 5 °C unter Rühren portionsweise mit 1,5 g (0,05 mol) 80 %igem Natriumhydrid versetzt. Bei Raumtemperatur gibt man nun 10,9 g (0,05 mol) 4,6-Dimethoxy-2-methylsulfonylpyrimidin zu, erwärmt auf 90 °C und rührt 6 h bei dieser Temperatur nach. Die so erhaltene Lösung wird in Wasser gegossen. Man extrahiert mit Methylenchlorid, wäscht mit Wasser, trocknet über Natriumsulfat und engt ein. Das Rohprodukt läßt sich durch Säulenchramatographie mit Toluol/Essigester weiter reinigen, wodurch 9,6 g eines Feststoffs vom Schmelzpunkt 98 - 102 °C erhalten wird.

Analog zu a) bzw. b) können beispielsweise auch die folgenden Verbindungen hergestellt werden:

6-(3,5-Dichlorphenyl)salicylsäuremthylester, $^1$H-NMR ($CDCl_3$: $\delta$ = 3,58 (s; 3H); 6,70 (d ; 1H); 7,05 (d; 1H); 7,10 (d; 2H); 7,25-7,40 (m; 2H); 10,85 (s; 1H).

6-(2,4-Dichlorphenyl)salicylsäuremthylester, $^1$H-NMR ($CDCl_3$ : $\delta$ = 3,58 (s; 3H); 6,65 (d; 1H); 7,0-7,5 (m; 5H); 11,1 (s; 1H).

2-(4,6-Dimethoxypyrimidin-2-yl)-oxi-6-(3,5-dichlorphenyl)benzoesäuremethylester (Verbindung Nr. 1.063), Schmp. 133-137 °C.

2-(4,6-Dimethoxypyrimidin-2-yl)-oxi-6-(2,4-dichlorphenyl)benzoesäuremethylester (Verbindung Nr. 1.024), Schmp. 99-100 °C.

Beispiel 2

Allgemeine Vorschrift zur Herstellung der Salicylsäurederivate der Formel I:

0,073 mol der jeweiligen aromatischen 2-Hydroxycarbonsäure werden in 320 ml getrocknetem Dimethylsulfoxid gelöst und portionsweise mit 16,4 g (0,146 mol) Kalium-tert.-butylat versetzt, wobei die Temperatur der Reaktionsmischung auf etwa 30 °C steigt. Man kühlt auf Raumtemperatur, gibt 16,0 g (0,073 mol) 4,6-Dimethoxy-2-methylsulfonylpyrimidin zu und rührt etwa 1 h bei Raumtemperatur nach. Die Reaktionsmischung wird in etwa 2 l kaltes Wasser gegossen, mit Salzsäure angesäuert und mit Methyl-tert.-butylether extrahiert. Nach üblicher Aufarbeitung kann das zurückbleibende Rohprodukt, wenn erforderlich, durch Verrühren mit einem geeigneten Lösungsmittel oder durch Chromatographie an Silica-Gel gereinigt werden.

Beispiel 3

Allgemeine Vorschrift zur Herstellung der Salicylsäurederivate der Formel I:

5,1 g Kaliumhydroxid und 0,08 mol der jeweiligen 2-Hydroxycarbonsäure werden in 80 ml Methanol gelöst, 10 min bei Raumtemperatur gerüht und im Vakuum eingeengt. Anschließend wird zum Trocknen wiederholt mit Toluol versetzt und dieses bei 50 °C im Vakuum verdampft. Das so erhaltene hellrote Pulver wird in 300 ml Dimethylsulfoxid aufgenommen und bei Raumtemperatur portionsweise mit 2,9 g 80 %igem Natriumhydrid versetzt, wobei eine Gasentwicklung auftritt. Wenn kein Gas mehr frei wird, tropft man eine Lösung von 17,4 g 4,6-Dimethoxy-2-methylsulfonylpyrimidin (oder eine äquivalente Menge des jeweiligen eingesetzten Pyridin, Pyrimidin- oder Triazinderivates) in 80 ml Dimethylsulfoxid zu und rührt 0,5 h nach. Man gießt in 2 l Wasser, neutralisiert mit Essigsäure und wäscht mit Methylenchlorid. Anschließend wird mit Salzsäure stark angesäuert und mehrmals mit Methyl-tert.-butylether extrahiert. Die organische Phase trocknet man über Natriumsulfat und verdampft das Lösungsmittel im Vakuum. Die zurückbleibende

Substanz kann durch Chromatograhie an Silica-Gel gereinigt werden.

Beispiel 4

Allgemeine Vorschrift zur Herstellung aromatischer Carbonsäureoximester oder ähnlicher Verbindungen der Formel I:

3,2 mmol der jeweiligen aromatischen 2-(4,6-Dimethoxypyrimidin-2-yl)oxycarbonsäure werden in 20 ml Diemthoxyethan vorgelegt und mit 3,2 mmol Natriumhydrid versetzt, wobei sofort eine Gasentwicklung auftritt. Es wird 1 h bei Raumtemperatur nachgerührt, auf 0 °C gekühlt und mit 3,5 mmol Oxalylchlorid versetzt. Man rührt bei 0 °C 1 h nach und verdampft dann zur Entfernung des überschüssigen Oxalylchlorids etwa 30 % des Lösungsmittel im Vakuum. Man gibt nun 4,2 mmol des jeweiligen Oxims oder einer vergleichbaren Hydroxy-Verbindung gelöst in 10 ml Dimethoxyethan und anschließend 3,2 mmol Pyridin bei 0 °C zu und erwärmt innerhalb von 1 h auf Raumtemperatur. Die Mischung wird in 120 ml kaltes Wasser gegossen und mit Methylenchlorid extrahiert. Die organische Phase wird über Natriumsulfat getrocknet und im Vakuum eingeengt. Die zurückbleibende Substanz kann durch Chromatographie an Silica-Gel weiter gereinigt werden.

Beispiel 5

2-(4,6-Dimethoxypyrimidin-2-yl)-oxi-6-(pyrazol-1-yl)benzoesäureethylester (Verbindung Nr. 2.004):

a) 2-Methoxy-6-(pyrazol-1-yl)-benzonitril:
Zu einer Lösung von 41,2 g (0,273 mol) 2-Methoxy-6-fluorbenzonitril (Herstellung: J. Heterocycl. Chem. 25, 1173 (1988) in 50 ml N,N-Dimethylethylenharnstoff werden unter Stickstoff bei 50 °C eine Lösung von 0,273 mol Natriumpyrazolid (hergestellt aus äquimolaren Mengen Pyrazol und Natriumhydrid) in 140 ml N,N-Dimethylethylenharnstoff zugetropft und 2 h bei 60 °C nachgerührt. Nach dem Abkühlen und Einrühren des Ansatzes in 4 l Eiswasser, Absaugen und Trocknen der ausgefallenen Kristalle erhält man 37,1 g des Produktes vom Schmelzpunkt 93-94 °C.
b) 2-Methoxy-6-(pyrazol-1-yl)benzoesäure-O-ethyliminoester:
In 68,0 g einer 30 %igen Lösung von trockenem HCl-Glas in Ethanol werden unter Rühren 37,1 g (0,186 mol) 2-Methoxy-6-(pyrazol-1-yl)benzonitril bei 0 °C unter Ausschluß von Feuchtigkeit eingetragen. Nach Verdünnen mit 20 ml Ethanol wird das Gemisch 48 h bei Raumtemperatur gerührt, in 500 ml Eiswasser gegeben und durch Zugabe von 2-normaler Natronlauge und dann gesättigter Natriumhydrogencarbonat-Lösung auf einen pH-Wert von 7 eingestellt. Nach dem Absaugen und Trocknen der ausgefallenen Kristalle erhält man 30,3 g des Produktes vom Schmelzpunkt 72-73 °C.
c) 2-Methoxy-6-(pyrazol-1-yl)benzoesäureethylester:
29,0 g (0,118 mol) 2-Methoxy-6-(pyrazol-1-yl)benzoesäure-O-ethyliminoester und 300 ml Salzsäure werden 16 h bei 50 °C gerührt. Nach dem Abkühlen wird das Reaktionsgemisch 3mal mit je 100 ml Methylenchlorid extrahiert. Nach dem Einengen des Extraktes erhält man 16,7 g Rückstand, der an Kieselgel mit Toluol/Essigsäureethylester (9:1) chromatographiert wird. Man erhält 13,4 g des Produktes vom Schmelzpunkt 159-163 °C.
d) 6-(Pyrazol-1-yl)-salicylsäureethylester:
Zu einer Lösung von 15,3 g (0,062 mol) 2-Methoxy-6-(pyrazol-1-yl)benzoesäureethylester in 140 ml Methylenchlorid werden bei 20 bis 25 °C 200 ml (0,2 mol) 1-molare Bortribromid-Lösung zugetrpft. Man rührt ca. 10 h bei Raumtemperatur und tropft dann bei 0 °C 160 ml Ethanol zu. Man rührt 15 min nach, entfernt dann das Lösungsmittel bei 30 °C weitergehend im Vakuum und verrührt den Rückstand mit 200 ml Wasser. Man extrahiert den Rückstand 3mal mit je 70 ml Diethylether, engt ein und chromatographiert das Rohprodukt an Kieselgel mit Toluol/Essigsäureethylester. Ausbeute: 8,7 g des Produktes als Öl. $^1$H-NMR (ausgewählte Signale): $\delta$ = 0,98 (t); 4,10 (q); 6,38; 6,90; 7,10; 7,45 (t); 7,60; 7,70; 10,70 (s).
Aus dieser Verbindung läßt sich 6-(Pyrazol-1-yl)-salicylsäure durch Hydrolyse mit verdünnter Natronlauge gewinnen (Schmelzpunkt: 175-179 °C).
e) 2-(4,6-Dimethoxypyrimidin-2-yl)-oxi-6-(pyrazol-1-yl)benzoesäuremethylester:
Zu 3,48 g (0,015 mol) 6-(Pyrazol-1-yl)-salicylsäureethylester gelöst in 25 ml getrocknetem Dimethylformamid werden bei 10 °C 0,46 g (0,015 mol) Natriumhydrid (80 %) gegeben und bei 30 °C 3 h nachgerührt. Dann werden 3,27 g (0,015 mol) 2-Methylsulfonyl-4,6-dimethoxypyrimidin zugegeben und ca. 12 h bei Raumtemperatur nachgerührt. Das Reaktionsgemisch wird in 500 ml Wasser gegeben, dem vorher 2,5 ml Orthophosphorsäure zugesetzt wurden. Das sich abscheidende Öl wird in Essigsäureethylester aufgenommen und über Natriumsulfat getrocknet. Nach dem Einengen erhält man 4,0 g eines kristallinen Rohproduktes, das durch Umkristallisieren aus Diethylether/Methyl-tert.-butylether weiter gereinigt wird. Schmelzpunkt 94 - 96 ° C.

Beispiel 6

2-(4,6-Dimethoxy)pyrimidin-2-yl)-oxi-6-(1,2,4-triazol-1-yl)benzoesäure (Verbindung Nr. 2.035)

a) 2-Methoxy-6(1,2,4-triazol-1-yl)benzonitril:
Zu einer Lösung von 0,21 mol Natriumtriazolid (hergestellt aus äquimolaren Mengen Triazol und Natriumhydrid) in 100 ml N,N-Dimethylformamid wird unter Stickstoff bei 45 bis 50 ° C eine Lösung von 30,2 g (0,20 mol) 2-Methoxy-6-fluorbenzonitril [Herstellung: J. Heterocycl. Chem. 25, 1173 (1988)] in 50 ml N,N-Dimethylformamid zugetropft und 2 h bei 50 ° C nachgerührt. Nach dem Verdampfen des Lösungsmittels im Vakuum wird der Rückstand mit 200 ml Eiswasser verrührt, mit etwas Eisessig auf pH6 eingestellt, abgesaugt und getrocknet. Nach Versetzen mit wenig Ether erhält man 35 g des Produktes von Schmelzpunkt 169 - 171 ° C (Z).
b) 2-Methoxy-6-(1,2,4-triazol-1-yl)benzamid:
15,0 g (0,075 mol) 2-Methoxy-6-(1,2,4-triazol-1-yl)benzonitril werden in 300 ml Wasser suspendiert, 9,6 (0,015 mol) 40 %ige Tetrabutylammoniumhydroxid-Lösung zugesetzt, 4 h am Rückfluß gekocht und im Vakuum eingedampft. Das Rohprodukt wird ohne weitere Reinigung weiter verseift (siehe C). Eine daraus isolierte Probe des obigen Produktes zeigt einen Schmelzpunkt von 140 - 150 ° C (Z).
c) 2-Methoxy-6-(1,2,4-triazol-1-yl)benzoesäure:
0,116 mol des obigen rohen 2-Methoxy-6-(1,2,4-triazol-1-yl)benzamids werden in einer Mischung aus 120 ml konz. Salzsäure und 60 ml Eisessig 5 h bei 100 ° C gerührt. Der Ansatz wird im Vakuum eingeengt und der Vorgang mit dem Rückstand wiederholt. Nach erneutem Einengen des Ansatzes wird der Rückstand in wenig Wasser gelöst und mit Natronlauge auf pH 3 eingestellt. Nach Zusatz von 30 bis 40 ml Ethanol wird der Niederschlag abgesaugt und im Methanol gelöst. Nach Trocknen und Einengen isoliert man 14,7 g der obigen Säure von Schmelzpunkt 200 - 202 ° C (Z).
d) 6-(1,2,4-Triazol-1-yl)salicylsäure:
9,2 g (0,042 mol) 2-Methoxy-6-(1,2,4-triazol-1-yl)benzoesäure und 50 ml Bromwasserstoffsäure (47 %ig) werden 5 h bei 100 ° C gerührt. Nach dem Verdampfen der Mineralsäure im Vakuum wird der Rückstand mit Ethanol aufgerührt und erneut im Vakuum eingeengt. Nach Ansteigen des Rückstands mit ca. 20 ml Wasser, Abstumpfen des pH auf 3,5 mit Natronlauge, Absaugen und Trocknen erhält man 4,6 g Rohprodukt, das nach Aufkochen mit 40 ml Essigester, Kühlen und Absaugen 3,5 g der obigen Salicylsäure vom Schmelzpunkt 214 bis 215 ° C (Z) liefert.
e) 2-(4,6-Dimethoxypyrimidin-2-yl)oxi-6-(1,2,4-triazol-1-yl)benzoesäure:
Zu 2,05 (0,01 mol) 6-(1,2,4-Triazol-1-yl)salicylsäure in 30 ml getrockneten Dimethylsulfoxid werden portionsweise 2,24 g (0,02 mol) Kalium-tert.-butylat gegeben und 1 h bei 40 ° C nachgerührt. Anschließend werden bei Raumtemperatur 2,18 g (0,01 mol) 2-Methylsulfonyl-4,6-dsimethoxypyrimidin zugegeben und ca. 20 h bei Raumtemperatur nachgerührt. Das Reaktionsgemisch wird in 450 ml Eiswasser gegeben, dem vorher 1 ml Orthophosphorsäure zugesetzt wurde. Nach Absaugen des Niederschlags, Waschen mit kaltem Wasser und Trocknet erhält man 2,85 g des obigen Produkts von Schmelzpunkt 158 bis 160 ° C.

Tabelle 1: Verbindungen I in denen A einen gegebenenfalls substituierten Phenylrest darstellt

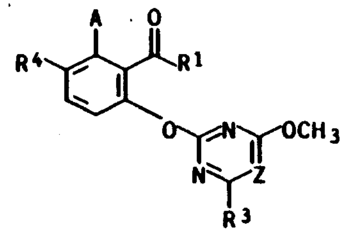

I

| Nr. | $R^1$ | $R^3$ | $R^4$ | A | Z | phys.Dat. Fp. [°C] |
|---|---|---|---|---|---|---|
| 1.001 | OH | $OCH_3$ | H | Phenyl | N | 120-125 |
| 1.002 | OH | $CH_3$ | H | Phenyl | N | |
| 1.003 | $OCH_3$ | $OCH_3$ | H | Phenyl | N | |
| 1.004 | OH | $OCH_3$ | H | Phenyl | CH | 185-195, Zers. |
| 1.005 | $OCH_3$ | $OCH_3$ | H | Phenyl | CH | 98-102 |
| 1.006 | 2-Propaniminoxy | $OCH_3$ | H | Phenyl | CH | |
| 1.007 | 1-Imidazolyl | $OCH_3$ | H | Phenyl | CH | |
| 1.008 | Methylthiomethoxy | $OCH_3$ | H | Phenyl | CH | |
| 1.009 | OH | $OCH_3$ | H | 2-Fluorphenyl | CH | 122-128 |
| 1.010 | $OC_2H_5$ | $OCH_3$ | H | 3-Fluorphenyl | CH | 52- 60 |
| 1.011 | OH | $OCH_3$ | H | 4-Fluorphenyl | CH | |
| 1.012 | OH | $OCH_3$ | H | 2,6-Difluorphenyl | CH | |
| 1.013 | OH | $OCH_3$ | H | 2,4-Difluorphenyl | CH | |
| 1.014 | OH | $OCH_3$ | H | 2-Fluor-4-trifluormethylphenyl | CH | |
| 1.015 | H | $OCH_3$ | H | 2,3-Difluorphenyl | CH | |

Tabelle 1 (Fortsetzung)

| Nr. | $R^1$ | $R^3$ | $R^4$ | A | Z | phys.Dat. Fp. [°C] |
|---|---|---|---|---|---|---|
| 1.016 | OH | $OCH_3$ | H | 2-Chlorphenyl | CH | 129-135 |
| 1.017 | $OC_2H_5$ | $OCH_3$ | H | 2-Iodphenyl | CH | |
| 1.018 | Methoxyethoxy | $OCH_3$ | H | 2-Bromphenyl | CH | |
| 1.019 | OH | $OCH_3$ | H | 2-Bromphenyl | CH | |
| 1.020 | OH | $OCH_3$ | H | 2-Chlorphenyl | N | |
| 1.021 | OH | $OCH_3$ | H | 2-Chlor-6-fluorphenyl | CH | |
| 1.022 | OH | $OCH_3$ | H | Pentafluorphenyl | CH | |
| 1.023 | OH | $OCH_3$ | H | 2,4-Difluorphenyl | CH | |
| 1.024 | $OCH_3$ | $OCH_3$ | H | 2,4-Dichlorphenyl | CH | 99-100 |
| 1.025 | OH | $OCH_3$ | H | 2,6-Dichlorphenyl | CH | |
| 1.026 | OH | $OCH_3$ | H | 2-Chlor-4-fluorphenyl | CH | |
| 1.027 | OH | $OCH_3$ | H | 3,5-Dichlorphenyl | CH | |
| 1.028 | 3-Dodecaniminoxy | $OCH_3$ | H | 3,5-Dichlorphenyl | CH | |
| 1.029 | OH | $OCH_3$ | H | 2-Methylphenyl | CH | 116-118 |
| 1.030 | $OC_2H_5$ | $OCH_3$ | H | 2-Methylphenyl | CH | |
| 1.031 | Ethylthioethoxy | $OCH_3$ | H | 2-Methylphenyl | CH | |
| 1.032 | 1-Imidazolyl | $OCH_3$ | H | 2-Methylphenyl | CH | |
| 1.033 | OH | $OCH_3$ | H | 2-Chlor-6-methylphenyl | CH | |
| 1.034 | OH | $OCH_3$ | H | 3-Methylphenyl | CH | 63- 67 |
| 1.035 | OH | $OCH_3$ | H | 4-Methylphenyl | CH | 50- 58 |
| 1.036 | OH | $OCH_3$ | H | 2,6-Dimethylphenyl | CH | |
| 1.037 | OH | $OCH_3$ | H | 3,5-Dimethylphenyl | CH | |
| 1.038 | OH | $OCH_3$ | H | 2,4,6-Trimethylphenyl | CH | 135-143 |

Tabelle 1 (Fortsetzung)

| Nr. | $R^1$ | $R^3$ | $R^4$ | A | Z | phys.Dat. Fp. [°C] |
|---|---|---|---|---|---|---|
| 1.039 | $OC_2H_5$ | $OCH_3$ | H | 2,4,6-Trimethylphenyl | CH | |
| 1.040 | OH | $OCH_3$ | H | 2,4-Dimethylphenyl | CH | |
| 1.041 | OH | $OCH_3$ | H | 2-Chlor-4-methylphenyl | CH | |
| 1.042 | OH | $OCH_3$ | H | 2,3,5-Trichlorphenyl | CH | |
| 1.043 | OH | $OCH_3$ | H | 4-Methoxyphenyl | CH | 82-116 |
| 1.044 | OH | $OCH_3$ | H | 2-Methoxyphenyl | CH | 145-149 |
| 1.045 | OH | $OCH_3$ | H | 4-Chlor-2-methoxyphenyl | CH | |
| 1.046 | OH | $OCH_3$ | H | 2-Trifluormethylphenyl | CH | |
| 1.047 | OH | $OCH_3$ | H | 2,3-Dimethyl-4-methoxyphenyl | CH | |
| 1.048 | OH | $OCH_3$ | H | 2-Dimethylamino-2-methylphenyl | CH | |
| 1.049 | OH | $OCH_3$ | H | 3-Cyanophenyl | CH | |
| 1.050 | $OC_2H_5$ | $OCH_3$ | H | 3-Nitrophenyl | CH | |
| 1.051 | OH | $OCH_3$ | H | 3-Phenoxyphenyl | CH | |
| 1.052 | OH | $OCH_3$ | H | 3-(3-Trifluormethylphenoxy)-phenyl | CH | |
| 1.053 | OH | $OCH_3$ | H | 3-Trifluormethylphenyl | CH | |
| 1.054 | $OCH_3$ | $OCH_3$ | H | 3-Trifluormethylphenyl | CH | |
| 1.055 | OH | $OCH_3$ | H | 3-Bromphenyl | CH | |
| 1.056 | OH | $OCH_3$ | H | 3-Chlorphenyl | CH | |
| 1.057 | OH | $OCH_3$ | H | 4-Bromphenyl | CH | 130-131 |
| 1.058 | OH | $OCH_3$ | H | 4-Chlorphenyl | CH | 132-134 |
| 1.059 | OH | $OCH_3$ | H | 4-Trifluormethylphenyl | CH | 130-131 |
| 1.060 | OH | $OCH_3$ | H | 3-(3,4-Dichlorphenoxy)phenyl | CH | |
| 1.061 | OH | $OCH_3$ | H | 4-Methylthiophenyl | CH | |

Tabelle 1 (Fortsetzung)

| Nr. | $R^1$ | $R^3$ | $R^4$ | A | Z | phys.Dat. Fp. [°C] |
|---|---|---|---|---|---|---|
| 1.062 | OH | $OCH_3$ | H | 4-t.-Butylphenyl | CH | 73- 75 |
| 1.063 | $OCH_3$ | $OCH_3$ | H | 3,5-Dichlorphenyl | CH | 133-137 |
| 1.064 | 2-(Ethoxyimino)-ethoxy | $OCH_3$ | H | Phenyl | CH | |
| 1.065 | 2-(Allyloxyimino)-ethoxy | $OCH_3$ | H | Phenyl | CH | |
| 1.066 | 2-(Benzyloxyimino)-ethoxy | $OCH_3$ | H | Phenyl | CH | |
| 1.067 | 2-(3-Chlorallyloxyimino)-phenoxy | $OCH_3$ | H | Phenyl | CH | |
| 1.068 | 2-(Ethoxyimino)propyloxy | $OCH_3$ | H | Phenyl | CH | |
| 1.069 | H | $OCH_3$ | H | Phenyl | CH | |
| 1.070 | Benzyloxy | $OCH_3$ | H | Phenyl | CH | |
| 1.071 | Propargyloxy | $OCH_3$ | H | Phenyl | CH | |
| 1.072 | Allyloxi | $OCH_3$ | H | Phenyl | CH | |
| 1.073 | Methoxymethoxy | $OCH_3$ | H | Phenyl | CH | |
| 1.074 | 2-Triflurethoxy | $OCH_3$ | H | Phenyl | CH | |
| 1.075 | Phenoxy | $OCH_3$ | H | Phenyl | CH | |
| 1.076 | 3-Dichlorallyloxy | $OCH_3$ | H | Phenyl | CH | |
| 1.077 | 1-Phenylethan-1-iminoxy | $OCH_3$ | H | Phenyl | CH | |
| 1.078 | OH | $CF_3$ | H | Phenyl | CH | 103-107 |
| 1.079 | OH | $OCHF_2$ | H | Phenyl | CH | |
| 1.080 | OK | $OCH_3$ | H | Phenyl | CH | 248-250 |
| 1.081 | ONa | $OCH_3$ | H | Phenyl | CH | 197-210 |
| 1.082 | OH | $SCH_3$ | H | Phenyl | N | |
| 1.083 | Cyclohexyloxy | $OCH_3$ | H | Phenyl | CH | |
| 1.084 | Cyclopentyloxy | $OCH_3$ | H | Phenyl | CH | |

Tabelle 1 (Fortsetzung)

| Nr. | $R^1$ | $R^3$ | $R^4$ | A | Z | phys.Dat. Fp. [°C] |
|---|---|---|---|---|---|---|
| 1.085 | $OCH_2COOC_2H_5$ | $OCH_3$ | H | Phenyl | CH | |
| 1.086 | 2-(Phenoxy)-ethoxy | $OCH_3$ | H | Phenyl | CH | |
| 1.087 | Phenylcarbonylmethoxy | $OCH_3$ | H | Phenyl | CH | |
| 1.088 | 4,5-Dichlorimidazol-1-yl | $OCH_3$ | H | Phenyl | CH | |
| 1.089 | Pyrazol-1-yl | $OCH_3$ | H | Phenyl | CH | |
| 1.090 | OH | $OCH_3$ | $CH_3$ | Phenyl | CH | 77- 81 |
| 1.091 | 2-(4-Methoxyphenoxy)-ethoxy | $OCH_3$ | H | Phenyl | CH | |
| 1.092 | OH | $OCH_3$ | H | 4-Ethoxycarbonylphenyl | CH | |
| 1.093 | $OCH_3$ | $OCH_3$ | H | 4-Fluorphenyl | CH | 166-168 |
| 1.094 | $OCH_3$ | $OCH_3$ | H | 4-Methoxyphenyl | CH | 112-121 |
| 1.095 | $OCH_3$ | $OCH_3$ | H | 3,5-Dichlorphenyl | CH | 130-139 |
| 1.096 | $OCH_3$ | $OCH_3$ | H | 4-Chlorphenyl | CH | 149-150 |
| 1.097 | $OCH_3$ | $OCH_3$ | H | 2-Fluorphenyl | CH | 111-114 |
| 1.098 | $OCH_3$ | $OCH_3$ | H | 3-Methylphenyl | CH | 70- 80 |
| 1.099 | $OCH_3$ | $OCH_3$ | H | 4-Methylphenyl | CH | 98-103 |
| 1.100 | $OCH_3$ | $OCH_3$ | H | 4-Nitrophenyl | CH | 124-135 |
| 1.101 | $OCH_3$ | $OCH_3$ | H | 3-Methoxyphenyl | CH | 73-112 |
| 1.102 | OH | $CF_3$ | H | 4-Methoxyphenyl | CH | 152-155 |
| 1.103 | OH | $CF_3$ | H | 4-Chlorphenyl | CH | 163-170 |
| 1.104 | $O^-mor^+$ a) | $OCH_3$ | H | 4-Chlorphenyl | CH | 148-149 |
| 1.105 | OH | $OCH_3$ | H | 4-Nitrophenyl | CH | 144-151 |

a) $mor^+$ = Morpholinyl-Ion
b) $atr^+$ = Aminotrialzolyl-Ion

Tabelle 1 (Fortsetzung)

| Nr. | $R^1$ | $R^3$ | $R^4$ | A | Z | phys.Dat. Fp. [°C] |
|---|---|---|---|---|---|---|
| 1.106 | OH | $OCH_3$ | H | 4-Phenoxyphenyl | CH | 67- 78 |
| 1.107 | OH | $OCH_3$ | H | 3-Methoxyphenyl | CH | 144-150 |
| 1.108 | OH | $OCH_3$ | H | 4-Carboxyphenyl | CH | 135-137 |
| 1.109 | OH | $OCH_3$ | H | 4-Trifluormethylphenyl | N | 138-144 |
| 1.110 | $OC_2H_5$ | $OCH_3$ | H | Phenyl | CH | 119-130 |
| 1.111 | OH | $OCH_3$ | H | 4-(4-Fluorphenoxy)phenyl | CH | 50- 56 |
| 1.112 | OH | $OCH_3$ | H | 4-Phenylphenyl | CH | 142-143 |
| 1.113 | OH | $OCH_3$ | H | 2-Nitrophenyl | CH | 156-160 |
| 1.114 | $O^-mor^+$ a) | $OCH_3$ | H | Phenyl | CH | 166-168 |
| 1.115 | $O^-atr^+$ b) | $OCH_3$ | H | Phenyl | CH | 99-105 |
| 1.116 | $O^-NH_4^+$ | $OCH_3$ | H | Phenyl | CH | 160-164 |
| 1.117 | $O^-Li^+$ | $OCH_3$ | H | Phenyl | CH | 215-217 |
| 1.118 | $O^-(0.5Ca^{2+})$ | $OCH_3$ | H | Phenyl | CH | 209-210 |
| 1.119 | $O^-N(C_4H_9)_4^+$ | $OCH_3$ | H | Phenyl | CH | Harz |

a) $mor^+$ = Morpholinyl-Ion

b) $atr^+$ = Aminotrialzolyl-Ion

Tabelle 2: Verbindungen I in denen A einen heterocyclischen oder bicyclischen Rest darstellt

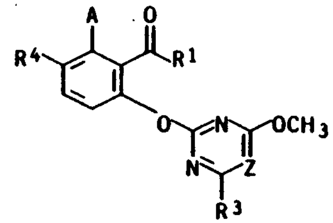

I

| Nr. | $R^1$ | $R^3$ | $R^4$ | A | Z | phys.Dat. Fp. [°C] |
|---|---|---|---|---|---|---|
| 2.001 | OH | $CH_3$ | H | Pyrazol-1-yl | N | |
| 2.002 | OH | $OCH_3$ | H | Pyrazol-1-yl | N | |
| 2.003 | OH | $OCH_3$ | H | Pyrazol-1-yl | CH | 120-125, Zers. |
| 2.004 | $OC_2H_5$ | $OCH_3$ | H | Pyrazol-1-yl | CH | 94- 96 |
| 2.005 | $OC_2H_5$ | $OCH_3$ | H | 4-Methylpyrazol-1-yl | CH | |
| 2.006 | $OC_2H_5$ | $OCH_3$ | H | 3,5-Dimethylpyrazol-1-yl | CH | |
| 2.007 | OH | $OCH_3$ | H | 3,5-Dimethylpyrazol-1-yl | CH | 149-152 |
| 2.008 | OH | $OCH_3$ | H | 3(5)-Methylpyrazol-1-yl | CH | 158-160 |
| 2.009 | OH | $OCH_3$ | H | 4-Methylpyrazol-1-yl | CH | 154-157 |
| 2.010 | $OC_2H_5$ | $OCH_3$ | H | 4-Chlorpyrazol-1-yl | CH | |
| 2.011 | OH | $OCH_3$ | H | 4-Chlorpyrazol-1-yl | CH | 134-138 |
| 2.012 | OH | $OCH_3$ | H | 4-Brompyrazol-1-yl | CH | |
| 2.013 | OH | $OCH_3$ | H | 4-Phenylpyrazol-1-yl | CH | 156-158 |
| 2.014 | $OC_2H_5$ | $OCH_3$ | H | 4-Phenylpyrazol-1-yl | CH | |
| 2.015 | OH | $OCH_3$ | H | 3,4,5-Trimethylpyrazol-1-yl | CH | 142-150 |

Tabelle 2 (Fortsetzung)

| Nr. | $R^1$ | $R^3$ | $R^4$ | A | Z | phys.Dat. Fp. [°C] |
|---|---|---|---|---|---|---|
| 2.016 | OH | $OCH_3$ | H | 4-Chlor-3,5-dimethylpyrazol-1-yl | CH | 71- 76 |
| 2.017 | OH | $OCH_3$ | H | 4-Isopropylpyrazol-1-yl | CH | |
| 2.018 | OH | $OCH_3$ | H | 3(5)-Phenylpyrazol-1-yl | CH | 100 (Z) |
| 2.019 | OH | $OCH_3$ | H | 3(5)-Methyl-5(3)-phenylpyrazol-1-yl | CH | 132 (Z) |
| 2.020 | OH | $OCH_3$ | H | 3,5-Bistrifluormethylpyrazol-1-yl | CH | |
| 2.021 | OH | $OCH_3$ | H | 4-Nitropyrazol-1-yl | CH | |
| 2.022 | Methylthiomethoxy | $OCH_3$ | H | Pyrazol-1-yl | CH | 88- 91 |
| 2.023 | Methoxyethoxy | $OCH_3$ | H | Pyrazol-1-yl | CH | |
| 2.024 | $OCH_2$-CO-$OC_2H_5$ | $OCH_3$ | H | Pyrazol-1-yl | CH | Öl |
| 2.025 | OH | $OCH_3$ | H | Imidazolyl-1-yl | CH | 199-201 |
| 2.026 | OH | $OCH_3$ | H | 2-Methylimidazol-1-yl | CH | |
| 2.027 | OH | $OCH_3$ | H | 4,5-Dimethylimidazol-1-yl | CH | |
| 2.028 | OH | $OCH_3$ | H | 2-Phenylimidazol-1-yl | CH | 164-165 |
| 2.029 | OH | $OCH_3$ | H | 4,5-Dichlorimidazol-1-yl | CH | |
| 2.030 | OH | $OCH_3$ | H | 2,4,5-Trichlorimidazol-1-yl | CH | |
| 2.031 | OH | $OCH_3$ | H | 2-Methyl-4,5-dichlorimidazol-1-yl | CH | |
| 2.032 | OH | $OCH_3$ | H | 2-Methyl-4,5-dibromimidazol-1-yl | CH | |
| 2.033 | OH | $OCH_3$ | H | 4(5)-Chlor-5(4)-methylimidazol-1-yl | CH | |
| 2.034 | OH | $OCH_3$ | H | 4(5)-Nitroimidazol-yl | CH | |
| 2.035 | OH | $OCH_3$ | H | [1,2,4]-Triazol-1-yl | CH | 158-160 |
| 2.036 | OH | $OCH_3$ | H | 3(5)-Methyl-[1,2,4]-triazol-1-yl | CH | 166-168 |
| 2.037 | OH | $OCH_3$ | H | 3(5)-Phenyl-[1,2,4]-triazol-1-yl | CH | 135-140 |
| 2.038 | OH | $OCH_3$ | H | 3,5-Dimethyl-[1,2,4]-triazol-1-yl | CH | 187-189 |

Tabelle 2 (Fortsetzung)

| Nr. | $R^1$ | $R^3$ | $R^4$ | A | Z | phys.Dat. Fp. [°C] |
|---|---|---|---|---|---|---|
| 2.039 | OH | $OCH_3$ | H | [1,2,3]-Triazol-1-yl | CH | |
| 2.040 | OH | $OCH_3$ | H | 4,5-Dimethyl-[1,2,3]-triazol-1-yl | CH | |
| 2.041 | OH | $OCH_3$ | H | 4(5)-Phenyl-[1,2,3]-triazol-1-yl | CH | |
| 2.042 | OH | $OCH_3$ | H | [1,2,3,4]-Tetrazol-1-yl | CH | |
| 2.043 | $OC_2H_5$ | $OCH_3$ | H | 1-Methylpyrazol-4-yl | CH | |
| 2.044 | $OC_2H_5$ | $OCH_3$ | H | 1-Phenylpyrazol-4-yl | CH | |
| 2.045 | OH | $OCH_3$ | H | 1-Phenylpyrazol-4-yl | CH | |
| 2.046 | $OC_2H_5$ | $OCH_3$ | H | 1,3,5-Trimethylpyrazol-4-yl | CH | |
| 2.047 | OH | $OCH_3$ | H | 1,3,5-Trimethylpyrazol-4-yl | CH | |
| 2.048 | OH | $OCH_3$ | H | 1-Methylpyrazol-4-yl | CH | |
| 2.049 | OH | $OCH_3$ | H | 1-Methylpyrazol-5-yl | CH | |
| 2.050 | OH | $OCH_3$ | H | 1-Phenylpyrazol-5-yl | CH | |
| 2.051 | OH | $OCH_3$ | H | 1-Methylpyrazol-3-yl | CH | |
| 2.052 | OH | $OCH_3$ | H | 1-Phenylpyrazol-3-yl | CH | |
| 2.053 | $OC_2H_5$ | $OCH_3$ | H | 1-Methylpyrazol-3-yl | CH | |
| 2.054 | OH | $OCH_3$ | H | 1,4-Dimethylpyrazol-3-yl | CH | |
| 2.055 | OH | $OCH_3$ | H | 5-Methyl-1-phenylpyrazol-3-yl | CH | |
| 2.056 | OH | $OCH_3$ | H | 1,5-Dimethylpyrazol-3-yl | CH | |
| 2.057 | OH | $OCH_3$ | H | 1,3-Dimethylpyrazol-4-yl | CH | |
| 2.058 | OH | $OCH_3$ | H | 1,5-Dimethylpyrazol-4-yl | CH | |
| 2.059 | OH | $OCH_3$ | H | 3-Methyl-1-phenylpyrazol-4-yl | CH | |
| 2.060 | OH | $OCH_3$ | H | 5-Methyl-1-phenylpyrazol-4-yl | CH | |
| 2.061 | OH | $OCH_3$ | H | 3,5-Dimethyl-1-phenylpyrazol-4-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.062 | OH | $OCH_3$ | H | 3-Methyl-1-phenylpyrazol-5-yl | CH | |
| 2.063 | OH | $OCH_3$ | H | 1,4-Dimethylpyrazol-5-yl | CH | |
| 2.064 | OH | $OCH_3$ | H | 1,3-Dimethylpyrazol-5-yl | CH | |
| 2.065 | OH | $OCH_3$ | H | 1-Methyl-[1,2,3]-triazol-5-yl | CH | |
| 2.066 | OH | $OCH_3$ | H | 1-Phenyl-[1,2,3]-triazol-5-yl | CH | |
| 2.067 | OH | $OCH_3$ | H | 1-Phenyl-[1,2,3]-triazol-4-yl | CH | |
| 2.068 | OH | $OCH_3$ | H | 5-Methyl-1-phenyl-[1,2,3]-triazol-4-yl | CH | |
| 2.069 | OH | $OCH_3$ | H | 5-Methyl-1-phenyl-[1,2,4]-triazol-3-yl | CH | |
| 2.070 | $OC_2H_5$ | $OCH_3$ | H | 1-Methylimidazol-2-yl | CH | |
| 2.071 | OH | $OCH_3$ | H | 1-Methylimidazol-2-yl | CH | |
| 2.072 | OH | $OCH_3$ | H | 1,4-Dimethylimidazol-5-yl | CH | |
| 2.073 | OH | $OCH_3$ | H | 1-Methyl-5-nitroimidazol-2-yl | CH | |
| 2.074 | OH | $OCH_3$ | H | 1-Methylimidazol-5-yl | CH | |
| 2.075 | OH | $OCH_3$ | H | 1-Phenylimidazol-5-yl | CH | |
| 2.076 | OH | $OCH_3$ | H | 2-Thienyl | CH | 70- 75 |
| 2.077 | OH | $OCH_3$ | H | 3-Thienyl | CH | |
| 2.078 | OH | $OCH_3$ | H | 2,3-Dichlor-4-thienyl | CH | |
| 2.079 | OH | $OCH_3$ | H | 2,5-Dichlor-3-thienyl | CH | |
| 2.080 | OH | $OCH_3$ | H | 2-Brom-5-thienyl | CH | |
| 2.081 | OH | $OCH_3$ | H | 4-Brom-2-thienyl | CH | |
| 2.082 | OH | $OCH_3$ | H | 3-Methyl-2-thienyl | CH | |
| 2.083 | OH | $OCH_3$ | H | 2-Chlor-5-thienyl | CH | |
| 2.084 | OH | $OCH_3$ | H | 2-Methyl-5-thienyl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.085 | OH | $OCH_3$ | H | 2-Nitro-5-thienyl | CH | |
| 2.086 | 1-Imidazolyl | $OCH_3$ | H | 2-Thienyl | CH | |
| 2.087 | 2-Propaniminoxi | $OCH_3$ | H | 3-Thienyl | CH | |
| 2.088 | Methylthiomethoxi | $OCH_3$ | H | 2-Thienyl | CH | |
| 2.089 | Ethoxicarbonylmethoxi | $OCH_3$ | H | 3-Thienyl | CH | |
| 2.090 | Allyloxi | $OCH_3$ | H | 2-Chlor-5-thienyl | CH | |
| 2.091 | Propargyloxi | $OCH_3$ | H | 2-Methyl-5-thienyl | CH | |
| 2.092 | OH | $OCH_3$ | H | Isoxazol-5-yl | CH | |
| 2.093 | OH | $OCH_3$ | H | 3-Methylisoxazol-5-yl | CH | |
| 2.094 | OH | $OCH_3$ | H | 3-Isopropylisoxazol-5-yl | CH | |
| 2.095 | OH | $OCH_3$ | H | 3-Phenylisoxazol-5-yl | CH | |
| 2.096 | OH | $OCH_3$ | H | 3-Methyl-4-chlorisoxazol-5-yl | CH | |
| 2.097 | OH | $OCH_3$ | H | 3-Methylisoxazol-4-yl | CH | |
| 2.098 | OH | $OCH_3$ | H | Isoxazol-4-yl | CH | |
| 2.099 | OH | $OCH_3$ | H | 3,5-Dimethylisoxazol-4-yl | CH | |
| 2.100 | $OC_2H_5$ | $OCH_3$ | H | 3-Isopropylisoxazol-5-yl | CH | |
| 2.101 | Benzyloxi | $OCH_3$ | H | 3-Isopropylisoxazol-5-yl | CH | |
| 2.102 | OH | $OCH_3$ | H | 2-Methyloxazol-4-yl | CH | |
| 2.103 | OH | $OCH_3$ | H | Oxazol-2-yl | CH | |
| 2.104 | OH | $OCH_3$ | H | 2-Methylthiazol-4-yl | CH | |
| 2.105 | OH | $OCH_3$ | H | 3-Isopropylisoxazol-5-yl | CH | |
| 2.106 | OH | $OCH_3$ | H | Thiazol-4-yl | CH | |
| 2.107 | OH | $OCH_3$ | H | 2-Benzylthiazol-4-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.108 | OH | $OCH_3$ | H | 5-Chlor-2-phenylthiazol-4-yl | CH | |
| 2.109 | OH | $OCH_3$ | H | Thiazol-2-yl | CH | |
| 2.110 | OH | $OCH_3$ | H | Thiazol-5-yl | CH | |
| 2.111 | OH | $OCH_3$ | H | 4-Methylthiazol-2-yl | CH | |
| 2.112 | OH | $OCH_3$ | H | 5-Methylthiazol-2-yl | CH | |
| 2.113 | OH | $OCH_3$ | H | 4-Phenylthiazol-2-yl | CH | |
| 2.114 | OH | $OCH_3$ | H | 4-Methylthiazol-5-yl | CH | |
| 2.115 | OH | $OCH_3$ | H | 2-Methylthiazol-5-yl | CH | |
| 2.116 | OH | $OCH_3$ | H | 2-Phenylthiazol-5-yl | CH | |
| 2.117 | $OC_2H_5$ | $OCH_3$ | H | 2-Phenylthiazol-5-yl | CH | |
| 2.118 | OH | $OCH_3$ | H | [1,3,4]Thiadiazol-2-yl | CH | |
| 2.119 | OH | $OCH_3$ | H | 5-Methyl-[1,3,4]thiadiazol-2-yl | CH | |
| 2.120 | OH | $OCH_3$ | H | 5-Phenyl-[1,3,4]thiadiazol-2-yl | CH | |
| 2.121 | OH | $OCH_3$ | H | 2-Pyridyl | CH | |
| 2.122 | OH | $OCH_3$ | H | 3-Pyridyl | CH | |
| 2.123 | OH | $OCH_3$ | H | 4-Pyridyl | CH | |
| 2.124 | OH | $OCH_3$ | H | 6-Methyl-2-pyridyl | CH | |
| 2.125 | OH | $OCH_3$ | H | 3-Chlor-5-pyridyl | CH | |
| 2.126 | OH | $OCH_3$ | H | 5-Chlor-2-pyridyl | CH | |
| 2.127 | OH | $OCH_3$ | H | Chinolin-2-yl | CH | |
| 2.128 | OH | $OCH_3$ | H | Chinolin-4-yl | CH | |
| 2.129 | OH | $OCH_3$ | H | Chinolin-8-yl | CH | |
| 2.130 | OH | $OCH_3$ | H | 7-Chlorchinolin-8-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.131 | OH | $OCH_3$ | H | 7-Chlor-3-methylchinolin-8-yl | CH | |
| 2.132 | OH | $OCH_3$ | H | 3,7-Dichlorchinolin-8-yl | CH | |
| 2.133 | OH | $OCH_3$ | H | 1-Naphthyl | CH | |
| 2.134 | OH | $OCH_3$ | H | 2-Naphthyl | CH | 132-138 |
| 2.135 | OH | $OCH_3$ | H | 2-Methyl-1-naphthyl | CH | |
| 2.136 | OH | $OCH_3$ | H | 2-Methoxy-1-naphthyl | CH | |
| 2.137 | OH | $OCH_3$ | H | 1-Chlor-4-naphthyl | CH | |
| 2.138 | $OC_2H_5$ | $OCH_3$ | H | 2-Naphthyl | CH | |
| 2.139 | $OC_2H_4SC_2H_5$ | $OCH_3$ | H | 2-Naphthyl | CH | |
| 2.140 | $OC_2H_5$ | $OCH_3$ | H | 2-Pyridyl | CH | |
| 2.141 | Allyloxi | $OCH_3$ | H | 3-Pyridyl | CH | |
| 2.142 | $OCH_3$ | $OCH_3$ | H | Pyrazol-1-yl | CH | |
| 2.143 | 2-Propaniminoxy | $OCH_3$ | H | Pyrazol-1-yl | CH | 117-120 |
| 2.144 | $OCH_3$ | $OCH_3$ | H | Indazol-1-yl | CH | |
| 2.145 | $OCH_3$ | $OCH_3$ | H | Benztriazol-1-yl | CH | |
| 2.146 | $OCH_3$ | $OCH_3$ | H | 3,5-Dimethylpyrazol-1-yl | CH | |
| 2.147 | $OCH_3$ | $OCH_3$ | H | 3(5)-Methylpyrazol-1-yl | CH | |
| 2.148 | $OCH_3$ | $OCH_3$ | H | 4-Methylpyrazol-1-yl | CH | |
| 2.149 | $OCH_3$ | $OCH_3$ | H | 4-Chlorpyrazol-1-yl | CH | |
| 2.150 | $OCH_3$ | $OCH_3$ | H | 4-Brompyrazol-1-yl | CH | |
| 2.151 | $OCH_3$ | $OCH_3$ | H | 4-Phenylpyrazol-1-yl | CH | |
| 2.152 | $OCH_3$ | $OCH_3$ | H | 3,4,5-Trimethylpyrazol-1-yl | CH | |
| 2.153 | $OCH_3$ | $OCH_3$ | H | 4-Chlor-3,5-dimethylpyrazol-1-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.154 | $OCH_3$ | $OCH_3$ | H | 4-Isopropylpyrazol-1-yl | CH | |
| 2.155 | $OCH_3$ | $OCH_3$ | H | 3(5)-Phenylpyrazol-1-yl | CH | |
| 2.156 | $OCH_3$ | $OCH_3$ | H | 3(5)-Methyl-5(3)-phenylpyrazol-1-yl | CH | |
| 2.157 | $OCH_3$ | $OCH_3$ | H | 3,5-Bistrifluormethylpyrazol-1-yl | CH | |
| 2.158 | $OCH_3$ | $OCH_3$ | H | 4-Nitropyrazol-1-yl | CH | |
| 2.159 | $OCH_3$ | $OCH_3$ | H | Imidazol-1-yl | CH | |
| 2.160 | $OCH_3$ | $OCH_3$ | H | 2-Methylimidazol-1-yl | CH | |
| 2.161 | $OCH_3$ | $OCH_3$ | H | 4,5-Dimethylimidazol-1-yl | CH | |
| 2.162 | $OCH_3$ | $OCH_3$ | H | 2-Phenylimidazol-1-yl | CH | |
| 2.163 | $OCH_3$ | $OCH_3$ | H | 4,5-Dichlorimidazol-1-yl | CH | |
| 2.164 | $OCH_3$ | $OCH_3$ | H | 2,4,5-Trichlorimidazol-1-yl | CH | |
| 2.165 | $OCH_3$ | $OCH_3$ | H | 2-Methyl-4,5-dichlorimidazol-1-yl | CH | |
| 2.166 | $OCH_3$ | $OCH_3$ | H | 2-Methyl-4,5-dibromimidazol-1-yl | CH | |
| 2.167 | $OCH_3$ | $OCH_3$ | H | 4(5)-Chlor-5(4)-methylimidazol-1-yl | CH | |
| 2.168 | $OCH_3$ | $OCH_3$ | H | 4(5)-Nitroimidazol-1-yl | CH | |
| 2.169 | $OCH_3$ | $OCH_3$ | H | [1,2,4]-Triazol-1-yl | CH | |
| 2.170 | $OCH_3$ | $OCH_3$ | H | 3(5)-Methyl-[1,2,4]-triazol-1-yl | CH | |
| 2.171 | $OCH_3$ | $OCH_3$ | H | 3(5)-Phenyl-[1,2,4]-triazol-1-yl | CH | |
| 2.172 | $OCH_3$ | $OCH_3$ | H | 3,5-Dimethyl-[1,2,4]-triazol-1-yl | CH | |
| 2.173 | $OCH_3$ | $OCH_3$ | H | [1,2,3]-Triazol-1-yl | CH | |
| 2.174 | $OCH_3$ | $OCH_3$ | H | 4,5-Dimethyl-[1,2,3]-triazol-1-yl | CH | |
| 2.175 | $OCH_3$ | $OCH_3$ | H | 4(5)-Phenyl-[1,2,3]-triazol-1-yl | CH | |
| 2.176 | $OCH_3$ | $OCH_3$ | H | [1,2,3,4]-Tetrazol-1-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.177 | $OCH_3$ | $OCH_3$ | H | 1-Phenylpyrazol-4-yl | CH | |
| 2.178 | $OCH_3$ | $OCH_3$ | H | 1,3,5-Trimethylpyrazol-4-yl | CH | |
| 2.179 | $OCH_3$ | $OCH_3$ | H | 1-Methylpyrazol-4-yl | CH | |
| 2.180 | $OCH_3$ | $OCH_3$ | H | 1-Methylpyrazol-5-yl | CH | |
| 2.181 | $OCH_3$ | $OCH_3$ | H | 1-Phenylpyrazol-5-yl | CH | |
| 2.182 | $OCH_3$ | $OCH_3$ | H | 1-Methylpyrazol-3-yl | CH | |
| 2.183 | $OCH_3$ | $OCH_3$ | H | 1-Phenylpyrazol-3-yl | CH | |
| 2.184 | $OCH_3$ | $OCH_3$ | H | 1,4-Dimethylpyrazol-3-yl | CH | |
| 2.185 | $OCH_3$ | $OCH_3$ | H | 5-Methyl-1-phenylpyrazol-3-yl | CH | |
| 2.186 | $OCH_3$ | $OCH_3$ | H | 1,5-Dimethylpyrazol-3-yl | CH | |
| 2.187 | $OCH_3$ | $OCH_3$ | H | 1,3-Dimethylpyrazol-4-yl | CH | |
| 2.188 | $OCH_3$ | $OCH_3$ | H | 1,5-Dimethylpyrazol-4-yl | CH | |
| 2.189 | $OCH_3$ | $OCH_3$ | H | 3-Methyl-1-phenylpyrazol-4-yl | CH | |
| 2.190 | $OCH_3$ | $OCH_3$ | H | 5-Methyl-1-phenylpyrazol-4-yl | CH | |
| 2.191 | $OCH_3$ | $OCH_3$ | H | 3,5-Dimethyl-1-phenylpyrazol-4-yl | CH | |
| 2.192 | $OCH_3$ | $OCH_3$ | H | 3-Methyl-1-phenylpyrazol-5-yl | CH | |
| 2.193 | $OCH_3$ | $OCH_3$ | H | 1,4-Dimethylpyrazol-5-yl | CH | |
| 2.194 | $OCH_3$ | $OCH_3$ | H | 1,3-Dimethylpyrazol-5-yl | CH | |
| 2.195 | $OCH_3$ | $OCH_3$ | H | 1-Methyl-[1,2,3]-triazol-5-yl | CH | |
| 2.196 | $OCH_3$ | $OCH_3$ | H | 1-Phenyl-[1,2,3]-triazol-5-yl | CH | |
| 2.197 | $OCH_3$ | $OCH_3$ | H | 1-Phenyl-[1,2,3]-triazol-4-yl | CH | |
| 2.198 | $OCH_3$ | $OCH_3$ | H | 5-Methyl-1-phenyl-[1,2,3]-triazol-4-yl | CH | |
| 2.199 | $OCH_3$ | $OCH_3$ | H | 5-Methyl-1-phenyl-[1,2,4]-triazol-3-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.200 | $OCH_3$ | $OCH_3$ | H | 1-Methylimidazol-2-yl | CH | |
| 2.201 | $OCH_3$ | $OCH_3$ | H | 1,4-Dimethylimidazol-5-yl | CH | |
| 2.202 | $OCH_3$ | $OCH_3$ | H | 1-Methyl-5-nitroimidazol-2-yl | CH | |
| 2.203 | $OCH_3$ | $OCH_3$ | H | 1-Methylimidazol-5-yl | CH | |
| 2.204 | $OCH_3$ | $OCH_3$ | H | 1-Phenylimidazol-5-yl | CH | |
| 2.205 | $OCH_3$ | $OCH_3$ | H | 2-Thienyl | CH | |
| 2.206 | $OCH_3$ | $OCH_3$ | H | 3-Thienyl | CH | |
| 2.207 | $OCH_3$ | $OCH_3$ | H | 2,3-Dichlor-4-thienyl | CH | |
| 2.208 | $OCH_3$ | $OCH_3$ | H | 2,5-Dichlor-3-thienyl | CH | |
| 2.209 | $OCH_3$ | $OCH_3$ | H | 2-Brom-5-thienyl | CH | |
| 2.210 | $OCH_3$ | $OCH_3$ | H | 4-Brom-2-thienyl | CH | |
| 2.211 | $OCH_3$ | $OCH_3$ | H | 3-Methyl-2-thienyl | CH | |
| 2.212 | $OCH_3$ | $OCH_3$ | H | 2-Chlor-5-thienyl | CH | |
| 2.213 | $OCH_3$ | $OCH_3$ | H | 2-Methyl-5-thienyl | CH | |
| 2.214 | $OCH_3$ | $OCH_3$ | H | 2-Nitro-5-thienyl | CH | |
| 2.215 | $OCH_3$ | $OCH_3$ | H | Isoxazol-5-yl | CH | |
| 2.216 | $OCH_3$ | $OCH_3$ | H | 3-Methylisoxazol-5-yl | CH | |
| 2.217 | $OCH_3$ | $OCH_3$ | H | 3-Isopropylisoxazol-5-yl | CH | |
| 2.218 | $OCH_3$ | $OCH_3$ | H | 3-Phenylisoxazol-5-yl | CH | |
| 2.219 | $OCH_3$ | $OCH_3$ | H | 3-Methyl-4-chlorisoxazol-5-yl | CH | |
| 2.220 | $OCH_3$ | $OCH_3$ | H | 3-Methylisoxazol-4-yl | CH | |
| 2.221 | $OCH_3$ | $OCH_3$ | H | Isoxazol-4-yl | CH | |
| 2.222 | $OCH_3$ | $OCH_3$ | H | 3,5-Dimethylisoxazol-4-yl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.223 | $OCH_3$ | $OCH_3$ | H | 2-Methyloxazol-4-yl | CH | |
| 2.224 | $OCH_3$ | $OCH_3$ | H | Oxazol-2-yl | CH | |
| 2.225 | $OCH_3$ | $OCH_3$ | H | 2-Methylthiazol-4-yl | CH | |
| 2.226 | $OCH_3$ | $OCH_3$ | H | 2-Phenylthiazol-4-yl | CH | |
| 2.227 | $OCH_3$ | $OCH_3$ | H | Thiazol-4-yl | CH | |
| 2.228 | $OCH_3$ | $OCH_3$ | H | 2-Benzylthiazol-4-yl | CH | |
| 2.229 | $OCH_3$ | $OCH_3$ | H | 5-Chlor-2-phenylthiazol-4-yl | CH | |
| 2.230 | $OCH_3$ | $OCH_3$ | H | Thiazol-2-yl | CH | |
| 2.231 | $OCH_3$ | $OCH_3$ | H | Thiazol-5-yl | CH | |
| 2.232 | $OCH_3$ | $OCH_3$ | H | 4-Methylthiazol-2-yl | CH | |
| 2.233 | $OCH_3$ | $OCH_3$ | H | 5-Methylthiazol-2-yl | CH | |
| 2.234 | $OCH_3$ | $OCH_3$ | H | 4-Phenylthiazol-2-yl | CH | |
| 2.235 | $OCH_3$ | $OCH_3$ | H | 4-Methylthiazol-5-yl | CH | |
| 2.236 | $OCH_3$ | $OCH_3$ | H | 2-Methylthiazol-5-yl | CH | |
| 2.237 | $OCH_3$ | $OCH_3$ | H | 2-Phenylthiazol-5-yl | CH | |
| 2.238 | $OCH_3$ | $OCH_3$ | H | [1,3,4]Thiadiazol-2-yl | CH | |
| 2.239 | $OCH_3$ | $OCH_3$ | H | 5-Methyl[1,3,4]thiadiazol-2-yl | CH | |
| 2.240 | $OCH_3$ | $OCH_3$ | H | 5-Phenyl[1,3,4]thiadiazol-2-yl | CH | |
| 2.241 | $OCH_3$ | $OCH_3$ | H | 2-Pyridyl | CH | |
| 2.242 | $OCH_3$ | $OCH_3$ | H | 3-Pyridyl | CH | |
| 2.243 | $OCH_3$ | $OCH_3$ | H | 4-Pyridyl | CH | |
| 2.244 | $OCH_3$ | $OCH_3$ | H | 6-Methyl-2-pyridyl | CH | |
| 2.245 | $OCH_3$ | $OCH_3$ | H | 3-Chlor-5-pyridyl | CH | |

Tabelle 2 (Fortsetzung)

| Nr. | $R^1$ | $R^3$ | $R^4$ | A | Z | phys.Dat. Fp. [°C] |
|---|---|---|---|---|---|---|
| 2.246 | $OCH_3$ | $OCH_3$ | H | 5-Chlor-2-pyridyl | CH | |
| 2.247 | $OCH_3$ | $OCH_3$ | H | Chinolin-2-yl | CH | |
| 2.248 | $OCH_3$ | $OCH_3$ | H | Chinolin-4-yl | CH | |
| 2.249 | $OCH_3$ | $OCH_3$ | H | Chinolin-8-yl | CH | |
| 2.250 | $OCH_3$ | $OCH_3$ | H | 7-Chlorchinolin-8-yl | CH | |
| 2.251 | $OCH_3$ | $OCH_3$ | H | 7-Chlor-3-methylchinolin-8-yl | CH | |
| 2.252 | $OCH_3$ | $OCH_3$ | H | 3,7-Dichlorchinolin-8-yl | CH | |
| 2.253 | $OCH_3$ | $OCH_3$ | H | 1-Naphthyl | CH | |
| 2.254 | $OCH_3$ | $OCH_3$ | H | 2-Naphthyl | CH | |
| 2.255 | $OCH_3$ | $OCH_3$ | H | 2-Methyl-1-naphthyl | CH | |
| 2.256 | $OCH_3$ | $OCH_3$ | H | 2-Methoxy-1-naphthyl | CH | |
| 2.257 | $OCH_3$ | $OCH_3$ | H | 1-Chlor-4-naphthyl | CH | |
| 2.258 | OH | $OCH_3$ | H | 3-Isopropylpyrazol-1-yl | CH | 55- 60 |
| 2.259 | OH | $OCH_3$ | H | 3-t.Butylpyrazol-1-yl | CH | 80 (Z) |
| 2.260 | $OC_2H_5$ | $OCH_3$ | H | Imidazol-1-yl | CH | Öl |
| 2.261 | $OC_2H_5$ | $OCH_3$ | H | 1,2,4-Triazol-1-yl | CH | Öl |
| 2.262 | OH | $OCH_3$ | H | Indazol-1-yl | CH | 95-100 |
| 2.263 | OH | $OCH_3$ | H | 3-n-Propylpyrazol-1-yl | CH | 48-50 |
| 2.264 | OH | $OCH_3$ | H | 3-Isobutylpyrazol-1-yl | CH | 88- 90 |
| 2.265 | OH | $OCH_3$ | H | 3-Neopentylpyrazol-1-yl | CH | 52- 55 |
| 2.266 | OH | $OCH_3$ | H | 3-Thiomethyl-1,2,4-triazol-1-yl | CH | 158- 60 |
| 2.267 | OH | $OCH_3$ | H | 5-Thiomethyl-3-methyl-1,2,4-triazol-1-yl | CH | 159- 61 |
| 2.268 | OH | $OCH_3$ | H | 3-Phenyl-5-methyl-1,2,4-triazol-1-yl | CH | 115-120 |

Tabelle 3: Salicylsäurederivate der Formel II'

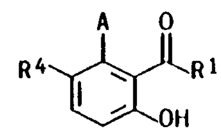

II'

| Nr. | $R^1$ | $R^4$ | A | phys.Dat. Fp. [°C] |
|---|---|---|---|---|
| 3.001 | OH | H | Pyrazol-1-yl | 175 - 179 |
| 3.002 | OH | H | 3,5-Dimethylpyrazol-1-yl | 182 (Z) |
| 3.003 | OH | H | 3(5)-Methylpyrazol-1-yl | 184 (Z) |
| 3.004 | OH | H | 4-Methylpyrazol-1-yl | 162 (Z) |
| 3.005 | OH | H | 4-Chlorpyrazol-1-yl | 175 (Z) |
| 3.006 | OH | H | 4-Brompyrazol-1-yl | |
| 3.007 | OH | H | 4-Phenylpyrazol-1-yl | 200-202 |
| 3.008 | OH | H | 3,4,5-Trimethylpyrazol-1-yl | 180 (Z) |
| 3.009 | OH | H | 4-Chlor-3,5-dimethylpyrazol-1-yl | 209-212 (Z) |
| 3.010 | OH | H | 4-Isopropylpyrazol-1-yl | |
| 3.011 | OH | H | 3(5)-Phenylpyrazol-1-yl | 212 (Z) |
| 3.012 | OH | H | 3(5)-Methyl-5(3)-phenylpyrazol-1-yl | 230-234 (Z). |

Tabelle 3 (Fortsetzung)

| Nr. | $R^1$ | $R^4$ | A | phys.Dat. Fp. [°C], Kp [°C/mbar] |
|---|---|---|---|---|
| 3.013 | OH | H | 3,5-Bistrifluormethylpyrazol-1-yl | |
| 3.014 | OH | H | 4-Nitropyrazol-1-yl | |
| 3.015 | OH | H | Imidazol-1-yl | 216 (Z) |
| 3.016 | OH | H | 2-Methylimidazol-1-yl | |
| 3.017 | OH | H | 4,5-Dimethylimidazol-1-yl | |
| 3.018 | OH | H | 2-Phenylimidazol-1-yl | |
| 3.019 | OH | H | 4,5-Dichlorimidazol-1-yl | 180 (Z) |
| 3.020 | OH | H | 2,4,5-Trichlorimidazol-1-yl | |
| 3.021 | OH | H | 2-Methyl-4,5-dichlorimidazol-1-yl | |
| 3.022 | OH | H | 2-Methyl-4,5-dibromimidazol-1-yl | |
| 3.023 | OH | H | 4(5)-Chlor-5(4)-methylimidazol-1-yl | |
| 3.024 | OH | H | 4(5)-Nitroimidazol-1-yl | |
| 3.025 | OH | H | [1,2,4]-Triazol-1-yl | 214-215 (Z) |
| 3.026 | OH | H | 3(5)-Methyl-[1,2,4]-triazol-1-yl | 210-211 (Z) |
| 3.027 | OH | H | 3(5)-Phenyl-[1,2,4]-triazol-1-yl | 225 (Z) |
| 3.028 | OH | H | 3,5-Dimethyl-[1,2,4]-triazol-1-yl | 255 (Z) |
| 3.029 | OH | H | [1,2,4]-Triazol-1-yl | |
| 3.030 | OH | H | 4,5-Dimethyl-[1,2,3]-triazol-1-yl | |
| 3.031 | OH | H | 4(5)-Phenyl-[1,2,3]-triazol-1-yl | |
| 3.032 | OH | H | [1,2,4]-Tetrazol-1-yl | |
| 3.033 | OH | H | 1-Phenyl-pyrazol-4-yl-pyrazol-4-yl | |
| 3.034 | OH | H | 1,3,5-Trimethylpyrazol-4-yl | |

Tabelle 3 (Fortsetzung)

| Nr. | $R^1$ | $R^4$ | A | phys.Dat. Fp. [°C], Kp [°C/mbar] |
|---|---|---|---|---|
| 3.035 | OH | H | 1-Methylpyrazol-4-yl | |
| 3.036 | OH | H | 1-Methylpyrazol-5-yl | |
| 3.037 | OH | H | 1-Phenylpyrazol-5-yl | |
| 3.038 | OH | H | 1-Methylpyrazol-3-yl | |
| 3.039 | OH | H | 1-Phenylpyrazol-3-yl | |
| 3.040 | OH | H | 1,4-Dimethylpyrazol-3-yl | |
| 3.041 | OH | H | 5-Methyl-1-phenyl-pyrazol-3-yl | |
| 3.042 | OH | H | 1,5-Dimethylpyrazol-3-yl | |
| 3.043 | OH | H | 1,3-Dimethylpyrazol-4-yl | |
| 3.044 | OH | H | 1,5-Dimethylpyrazol-4-yl | |
| 3.045 | OH | H | 3-Methyl-1-phenylpyrazol-4-yl | |
| 3.046 | OH | H | 5-Methyl-1-phenylpyrazol-4-yl | |
| 3.047 | OH | H | 3,5-Dimethyl-1-phenylpyrazol-4-yl | |
| 3.048 | OH | H | 3-Methyl-1-phenylpyrazol-5-yl | |
| 3.049 | OH | H | 1,4-Dimethylpyrazol-5-yl | |
| 3.050 | OH | H | 1,3-Dimethylpyrazol-5-yl | |
| 3.051 | OH | H | 1-Methyl-[1,2,3]-triazol-5-yl | |
| 3.052 | OH | H | 1-Phenyl-[1,2,4]-triazol-5-yl | |
| 3.053 | OH | H | 1-Phenyl-[1,2,3]-triazol-4-yl | |
| 3.054 | OH | H | 5-Methyl-1-phenyl-[1,2,3]-triazol-4-yl | |
| 3.055 | OH | H | 5-Methyl-1-phenyl-[1,2,4]-triazol-3-yl | |
| 3.056 | OH | H | 1-Methylimidazol-2-yl | |

Tabelle 3 (Fortsetzung)

| Nr. | $R^1$ | $R^4$ | A | phys.Dat. Fp. [°C], Kp [°C/mbar] |
|---|---|---|---|---|
| 3.057 | OH | H | 1,4-Dimethylimidazol-5-yl | |
| 3.058 | OH | H | 1-Methyl-5-nitroimidazol-2-yl | |
| 3.059 | OH | H | 1-Methylimidazol-5-yl | |
| 3.060 | OH | H | 1-Phenylimidazol-5-yl | |
| 3.061 | OH | H | 2-Thienyl | |
| 3.062 | OH | H | 3-Thienyl | |
| 3.063 | OH | H | 2,3-Dichlor-4-thienyl | |
| 3.064 | OH | H | 2,5-Dichlor-3-thienyl | |
| 3.065 | OH | H | 2-Brom-5-thienyl | |
| 3.066 | OH | H | 4-Brom-2-thienyl | |
| 3.067 | OH | H | 3-Methyl-2-thienyl | |
| 3.068 | OH | H | 2-Chlor-5-thienyl | |
| 3.069 | OH | H | 2-Methyl-5-thienyl | |
| 3.070 | OH | H | 2-Nitro-5-thienyl | |
| 3.071 | OH | H | Isoxazol-5-yl | |
| 3.072 | OH | H | 3-Methylisoxazol-5-yl | |
| 3.073 | OH | H | 3-Isopropylisoxazol-5-yl | |
| 3.074 | OH | H | 3-Phenylisoxazol-5-yl | |
| 3.075 | OH | H | 3-Methyl-4-chlorisoxazol-5-yl | |
| 3.076 | OH | H | 3-Methylisoxazol-4-yl | |
| 3.077 | OH | H | Isoxazol-4-yl | |
| 3.078 | OH | H | 3,5-Dimethylisoxazol-4-yl | |

Tabelle 3 (Fortsetzung)

| Nr. | $R^1$ | $R^4$ | A | phys.Dat. Fp. [°C], Kp [°C/mbar] |
|---|---|---|---|---|
| 3.079 | OH | H | 2-Methyloxazol-4-yl | |
| 3.080 | OH | H | Oxazol-2-yl | |
| 3.081 | OH | H | 2-Methylthiazol-4-yl | |
| 3.082 | OH | H | 2-Phenylthiazol-4-yl | |
| 3.083 | OH | H | Thiazol-4-yl | |
| 3.084 | OH | H | 2-Benzylthiazol-4-yl | |
| 3.085 | OH | H | 5-Chlor-2-phenylthiazol-4-yl | |
| 3.086 | OH | H | Thiazol-2-yl | |
| 3.087 | OH | H | Thiazol-5-yl | |
| 3.088 | OH | H | 4-Methylthiazol-2-yl | |
| 3.089 | OH | H | 5-Methylthiazol-2-yl | |
| 3.090 | OH | H | 4-Phenylthiazol-2-yl | |
| 3.091 | OH | H | 4-Methylthiazol-5-yl | |
| 3.092 | OH | H | 2-Methylthiazol-5-yl | |
| 3.093 | OH | H | 2-phenylthiazol-5-yl | |
| 3.094 | OH | H | [1,3,4]-Thiadiazol-2-yl | |
| 3.095 | OH | H | 5-Methyl-[1,3,4]-thiadiazol-2-yl | |
| 3.096 | OH | H | 5-Phenyl-[1,3,4]-thiadiazol-2-yl | |

Tabelle 3 (Fortsetzung)

| Nr. | $R^1$ | $R^4$ | A | phys.Dat. Fp. [°C], Kp [°C/mbar] |
|---|---|---|---|---|
| 3.097 | OH | H | Chinolin-2-yl | |
| 3.098 | OH | H | Chinolin-4-yl | |
| 3.099 | OH | H | Chinolin-8-yl | |
| 3.100 | OH | H | 7-Chlorchinolin-8-yl | |
| 3.101 | OH | H | 7-Chlor-3-methylchinolin-8-yl | |
| 3.102 | OH | H | 3,7-Dichlorchinolin-8-yl | |
| 3.103 | OH | H | 1-Naphthyl | |
| 3.104 | OH | H | 2-Naphthyl | 159-176 |
| 3.105 | OH | H | 2-Methyl-1-naphthyl | |
| 3.106 | OH | H | 2-Methoxy-1-naphthyl | |
| 3.107 | OH | H | 1-Chlor-4-naphthyltriazol-5-yl | |
| 3.108 | $OCH_3$ | H | Pyrazol-1-yl | Öl |
| 3.109 | $OCH_3$ | H | 2-Naphthyl | 175-195/0,57 |
| 3.110 | $OCH_3$ | H | 2-Thienyl | 118-160/0,4 |
| 3.111 | OH | H | 3-Isopropyl-pyrazol-1-yl | 160- 63 |
| 3.112 | OH | H | 3(5)-Methylthio-1,2,4-triazol-1-yl | 215 (Z) |
| 3.113 | OH | H | 3(5)-Methylthio-5(3)-methyl-1,2,4-triazol-1-yl | 130 (Z) |
| 3.114 | OH | H | 3(5)-Phenyl-5(3)-methyl-1,2,4-triazol-1-yl | 240 (Z) |

Anwendungsbeispiele zur herbiziden Wirkung

Die herbizide Wirkung der Carbonsäurederivate der Formel I ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge für die Nachauflaufbehandlung betrug 0,5 kg Wirkstoff/ha a.S. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35 °C) und für solche gemäßigter Klimate 10 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung und normaler Wachstumsverlauf.

Die in den Gewächshausversuchen verwendeten Pflanzen setzten sich aus folgenden Arten zusammen:

| Abkürzung | Lateinischer Name | Deutscher Name |
|---|---|---|
| BROIN | Bromus inermis | unbegrannte Trespe |
| ECHCG | Echinochloa crus-galli | Hühnerhirse |
| IPOSS | Impomoea spp. | Prunkwindearten |

Mit 0,5 kg/ha a.S. im Nachauflaufverfahren eingesetzt, lassen sich mit dem Beispiel 1.004 unerwünschte Pflanzen sehr gut bekämpfen.

## Patentansprüche

**1.** Salicylaldehyd- und Salicylsäurederivate und deren Schwefelanaloge der Formel I,

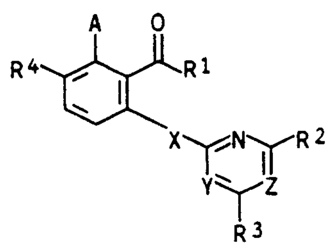

I

in der die Substituenten folgende Bedeutung haben:

$R^1$ Wasserstoff

eine Succinyliminooxygruppe;

ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

einen Rest $-OR^5$ oder einen Rest $ON{=}CR^6R^7$, worin

$R^5$

Wasserstoff, ein Alkalimetallkation, das Äquivalent eines Erdalkalimetallkations oder ein organisches Ammoniumion;
eine $C_3$-$C_{12}$-Cycloalkylgruppe, welche ein bis drei $C_1$-$C_4$-Alkylreste tragen kann;
eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, $C_3$-$C_{12}$-Cycloalkyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
eine $C_1$-$C_{10}$-Alkylgruppe, welche ein bis fünf Halogenatome tragen kann und einen der folgenden Reste trägt: ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
eine $C_2$-$C_6$-Alkylgruppe, welche in der 2-Position einen der folgenden Reste trägt: $C_1$-$C_6$-Alkoxyimino, $C_3$-$C_6$-Alkenyloxyimino, $C_3$-$C_6$-Halogenalkenyloxyimino oder Benzyloxyimino;
eine $C_3$-$C_6$-Alkenyl- oder eine $C_3$-$C_6$-Alkinylgruppe, wobei diese Gruppen ihrerseits ein bis fünf Halogenatome tragen können;
unsubstituiertes oder ein- bis dreifach durch $C_1$-$C_4$-Alkyl oder $C_1$-$C_4$-Alkoxy substituiertes oder ein- bis fünffach durch Halogen substituiertes Phenyl;
bedeutet, und
$R^6$ und $R^7$
$C_1$-$C_{20}$-Alkyl, welches einen Phenylrest, eine $C_1$-$C_4$-Alkoxy- und/oder eine $C_1$-$C_4$-Alkylthiogruppe tragen kann, Phenyl oder gemeinsam eine $C_3$-$C_{12}$-Alkylenkette, welche ein bis drei $C_1$-$C_3$-Alkylgruppen tragen kann, bedeuten;

$R^2$, $R^3$ $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
X ein Sauerstoff- oder Schwefelatom;
Y,Z ein Stickstoffatom oder eine Methingruppe =CH-;
$R^4$ Wasserstoff, Halogen, $C_1$-$C_4$-Alkyl, Cyano oder $C_1$-$C_4$-Halogenalkyl;
A einen gegebenenfalls ein bis dreifach substituierten, im Fall von Halogen als Substituent ein bis fünffach substituierten Phenylrest

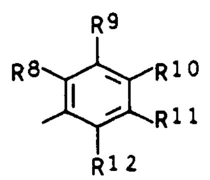

worin
$R^8$ - $R^{12}$ Wasserstoff, Halogen, Cyano, Nitro;
eine $C_3$-$C_6$-Alkenyl-, $C_3$-$C_6$-Alkenyloxy-, $C_3$-$C_6$-Alkinyloxy- oder eine $C_3$-$C_6$-Alkinylgruppe, wobei diese Gruppen ihrerseits ein bis fünf Halogenatome tragen können;
Di-$C_1$-$C_4$-alkylamino, $C_3$-$C_8$-Cycloalkyl, welches ein bis drei $C_1$-$C_4$-Alkylreste tragen kann;
$C_1$-$C_{10}$-Alkoxycarbonyl, $C_1$-$C_4$-Alkylthio;
eine Phenoxygruppe, wobei der aromatische Rest ein bis fünf Halogenatome und/oder ein bis drei der folgenden Reste tragen kann, $C_1$-$C_4$-Alkyl, $C_1$-$C_4$-Halogenalkyl, $C_1$-$C_4$-Halogenalkoxy, $C_1$-$C_4$-Alkoxy, $C_1$-$C_4$-Alkylthio;
eine $C_1$-$C_{10}$-Alkyl- oder Alkoxygruppe, welche ein bis fünf Halogenatome und/oder einen der folgenden Reste tragen kann: $C_1$-$C_4$-Alkoxy, $C_1$-$C_4$-Alkylthio, Phenyl oder Phenoxy, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Halogenalkoxy, $C_1$-$C_4$-Alkylthio;
bedeuten;
einen 5-gliedrigen Heteroaromaten, mit zwei bis vier Stickstoffatomen oder ein bis zwei Stickstoffatomen sowie zusätzlich einem Schwefel- oder Sauerstoffatom im Ring, welcher ein

bis drei Halogenatome und/oder einen bis drei der folgenden Reste tragen kann: Nitro, Cyano, $C_1$-$C_4$-Alkyl, $C_1$-$C_4$-Alkylthio, $C_1$-$C_4$-Halogenalkyl oder Phenyl, das unsubstituiert oder durch ein bis drei Halogenatome und/oder ein bis drei Methylgruppen substituiert ist;
einen Thienylrest, der ein bis drei Halogenatome und/oder einen bis drei der folgenden Reste tragen kann: $C_1$-$C_4$-Alkyl, $C_1$-$C_2$-Halogenalkyl oder Nitro;
einen Pyridylrest, der ein bis drei Halogenatome und/oder einen bis drei der folgenden Reste tragen kann: $C_1$-$C_4$-Alkyl, $C_1$-$C_2$-Halogenalkyl oder Nitro;
einen Naphthyl-, Chinolin-, Indazolyl- oder Benztriazolylrest, welcher jeweils ein bis drei Halogenatome und/oder einen bis drei der folgenden Reste tragen kann: $C_1$-$C_4$-Alkyl oder $C_1$-$C_2$-Halogenalkyl,

sowie umweltverträgliche Salze der Verbindungen I, beispielsweise Alkalimetallsalze, Erdalkalimetallsalze, Mangan-, Kupfer-, Zink- oder Eisensalze sowie Ammonium-, Phosphonium-, Sulfonium- oder Sulfoxoniumsalze.

2. Verfahren zur Herstellung der Verbindungen der Formel I gemäß Anspruch 1, dadurch gekennzeichnet, daß man eine entsprechendes Salicylsäure- oder (Thio)salicylsäurederivat der Formel II

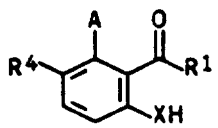

II

in an sich bekannter Weise mit einem Heterocyclus der Formel III,

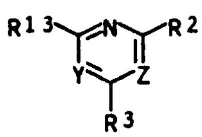

III

worin $R^{13}$ für eine nucleofuge Abgangsgruppe steht, in Gegenwart einer anorganischen oder organischen Base umsetzt.

3. Verfahren zur Herstellung der Verbindungen der Formel I gemäß Anspruch 1, dadurch gekennzeichnet, daß man die freie Salicylsäure bzw. ihre Schwefelanalogen der Formel I'

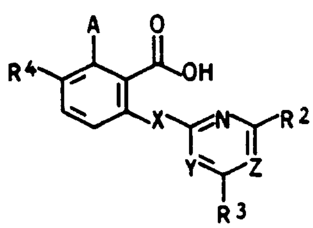

I'

zunächst in an sich bekannter Weise in das Halogenid oder eine andere aktivierte Form der Carbonsäure überführt und diese dann gegebenenfalls in Gegenwart einer anorganischen oder organischen Base mit einem gegebenenfalls substituierten Alkohol, einem Azol, einem Oxim oder N-Hydroxysuccinylimin umsetzt.

4. Herbizides Mittel, enthaltend eine Verbindung der Formel I gemäß Anspruch 1.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

**6.** Verwendung von Salicylaldehyd- und Salicylsäurederivaten und deren Schwefelanalogen der Formel I gemäß Anspruch 1 zur Bekämpfung unerwünschten Pflanzenwuchses.

**7.** Mittel zur Beeinflussung des Pflanzenwachstums, enthaltend ein Salicylaldehyd- oder Salicylsäurederivat der Formel I gemäß Anspruch 1.

**8.** Verfahren zur Regulierung des Pflanzenwachstums, dadurch gekennzeichnet, daß man eine regulativ wirksame Menge eines Salicylaldehyd- bzw. Salicylsäurederivats der allgemeinen Formel Ia gemäß Anspruch 1 auf die Samen, die Pflanzen und/oder deren Lebensraum einwirken läßt.

**9.** Salicylsäurederivate der allgemeinen Formel II'

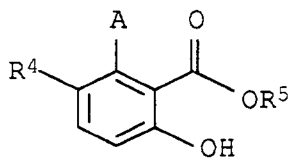

II'

in der die Substituenten folgende Bedeutung haben:

$R^5$ Wasserstoff, ein Alkalimetallkation, das Äquivalent eines Erdalkalimetallkations, ein organisches Ammoniumion;
eine $C_1$-$C_{10}$-Alkylgruppe, welche ein bis fünf Halogenatome und/oder einen der folgenden Reste tragen kann: $C_1$-$C_4$-Alkoxy, $C_1$-$C_4$-Alkylthio, Phenyl, Phenoxy, wobei die Phenylreste jeweils ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

$R^4$ Wasserstoff, ein Halogenatom, $C_1$-$C_4$-Alkyl, Cyano oder $C_1$-$C_4$-Halogenalkyl;

A einen 5-gliedrigen Heteroaromaten, mit zwei bis vier Stickstoffatomen oder ein bis zwei Stickstoffatomen sowie zusätzlich einem Schwefel- oder Sauerstoffatom im Ring, welcher ein bis drei Halogenatome und/oder einen bis drei der folgenden Reste tragen kann: Nitro, Cyano, $C_1$-$C_4$-Alkyl, $C_1$-$C_4$-Alkylthio, $C_1$-$C_4$-Halogenalkyl oder Phenyl, das unsubstituiert oder durch ein bis drei Halogenatome und/oder ein bis drei Methylgruppen substituiert ist;
einen Thienylrest, der ein bis drei Halogenatome und/oder einen bis drei der folgenden Reste tragen kann: $C_1$-$C_4$-Alkyl, $C_1$-$C_2$-Halogenalkyl oder Nitro;
einen Naphthyl-, Chinolin-, Indazolyl- oder Benztriazolylrest, welcher jeweils ein bis drei Halogenatome und/oder einen bis drei der folgenden Reste tragen kann: $C_1$-$C_4$-Alkyl oder $C_1$-$C_2$-Halogenalkyl.

**10.** Salicylaldehyd- und Salicylsäurederivate der Formel I gemäß Anspruch 1, in der $R^2$ $OCH_3$, X Sauerstoff, Y Stickstoff, Z die Methingruppe und A sowie die Reste $R^1$, $R^3$ und $R^4$ die in Anspruch 1 genannte Bedeutung haben.

**11.** Salicylsäurederivat der Formel I gemäß Anspruch 1, in der $R^1$ OH, $R^2$ und $R^3$ $OCH_3$, $R^4$ Wasserstoff, X Sauerstoff, Y Stickstoff, Z die Methingruppe und A Phenyl bedeuten.

## Claims

1. A salicylaldehyde derivative or salicylic acid derivative or sulfur analog thereof of the formula I

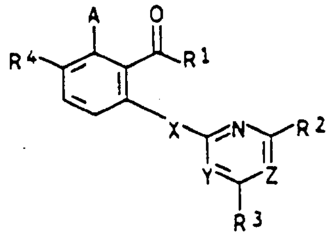

I

where
$R^1$ is hydrogen;
succinyliminooxy;
a 5-membered heteroaromatic radical containing from one to three nitrogen atoms which may carry from one to four halogen atoms and/or one or two of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
a radical -$OR^5$ or a radical $ON=CR^6R^7$, where
$R^5$ is hydrogen, an alkali metal cation, one equivalent of an alkaline earth metal cation or an organic ammonium ion;
$C_3$-$C_{12}$-cycloalkyl which may carry from one to three $C_1$-$C_4$-alkyl radicals;
$C_1$-$C_{10}$-alkyl which may carry from one to five halogen atoms and/or one of the following radicals: $C_1$-$C_4$-alkoxy, $C_1$-$C_4$-alkylthio, cyano, $C_1$-$C_8$-alkylcarbonyl, $C_1$-$C_8$-alkoxycarbonyl, $C_3$-$C_{12}$-cycloalkyl, phenyl, phenoxy or phenylcarbonyl, where the aromatic radicals may in turn carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
$C_1$-$C_{10}$-alkyl which may carry from one to five halogen atoms and carries one of the following radicals: a 5-membered heteroaromatic radical containing from one to three nitrogen atoms which may carry from one to four halogen atoms and/or one or two of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
$C_2$-$C_6$-alkyl which carries one of the following radicals in the 2-position: $C_1$-$C_6$-alkoxyimino, $C_3$-$C_6$-alkenyloxyimino, $C_3$-$C_6$-haloalkenyloxyimino or benzyloxyimino;
$C_3$-$C_6$-alkenyl or $C_3$-$C_6$-alkynyl, where these groups may in turn carry from one to five halogen atoms;
phenyl which is unsubstituted or monosubstituted to trisubstituted by $C_1$-$C_4$-alkyl or by $C_1$-$C_4$-alkoxy or monosubstituted to pentasubstituted by halogen;
$R^6$ and $R^7$ are each $C_1$-$C_{20}$-alkyl which may carry phenyl, $C_1$-$C_4$-alkoxy and/or $C_1$-$C_4$-alkylthio, or are each phenyl or together form a $C_3$-$C_{12}$-alkylene chain which may carry from one to three $C_1$-$C_3$-alkyl groups;
$R^2$ and $R^3$ are each $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
X is oxygen or sulfur;
Y and Z are each nitrogen or a methine group =CH-; $R^4$ is hydrogen, halogen, $C_1$-$C_4$-alkyl, cyano or $C_1$-$C_4$-haloalkyl;
A is an unsubstituted or monosubstituted to trisubstituted, or with halogen as substituent, monosubstituted to pentasubstituted phenyl radical

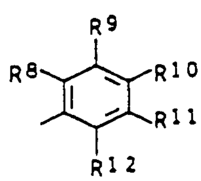

where $R^8$ to $R^{12}$ are each hydrogen, halogen, cyano or nitro;
$C_3$-$C_6$-alkenyl, $C_3$-$C_6$-alkenyloxy, $C_3$-$C_6$-alkynyloxy or $C_3$-$C_6$-alkynyl, where these groups may in turn

carry from one to five halogen atoms;
di-$C_1$-$C_4$-alkylamino or $C_3$-$C_8$-cycloalkyl which may carry from one to three $C_1$-$C_4$-alkyl radicals;
$C_1$-$C_{10}$-alkoxycarbonyl or $C_1$-$C_4$-alkylthio;
phenoxy where the aromatic radical may carry from one to five halogen atoms and/or from one to three of the following radicals: $C_1$-$C_4$-alkyl, $C_1$-$C_4$-haloalkyl, $C_1$-$C_4$-haloalkoxy, $C_1$-$C_4$-alkoxy or $C_1$-$C_4$-alkylthio; and
$C_1$-$C_{10}$-alkyl or alkoxy which may carry from one to five halogen atoms and/or one of the following radicals: $C_1$-$C_4$-alkoxy, $C_1$-$C_4$-alkylthio, phenyl or phenoxy, where the aromatic radicals may in turn carry from one to five halogen atoms and/or from one to three of the following radicals: $C_1$-$C_4$-alkyl, $C_1$-$C_4$-haloalkyl, $C_1$-$C_4$-haloalkoxy or $C_1$-$C_4$-alkylthio;
or A is a 5-membered heteroaromatic radical having from two to four nitrogen atoms or one or two nitrogen atoms and additionally one sulfur or oxygen atom in the ring, which may carry from one to three halogen atoms and/or from one to three of the following radicals: nitro, cyano, $C_1$-$C_4$-alkyl, $C_1$-$C_4$-alkylthio, $C_1$-$C_4$-haloalkyl or phenyl which is unsubstituted or substituted by from one to three halogen atoms and/or from one to three methyl groups;
thienyl which may carry from one to three halogen atoms and/or from one to three of the following radicals: $C_1$-$C_4$-alkyl, $C_1$- or $C_2$-haloalkyl or nitro;
pyridyl which may carry from one to three halogen atoms and/or from one to three of the following radicals: $C_1$-$C_4$-alkyl, $C_1$- or $C_2$-haloalkyl or nitro;
a naphthyl, quinolyl, indazolyl or benzotriazolyl radical, each of which may carry from one to three halogen atoms and/or from one to three of the following radicals: $C_1$-$C_4$-alkyl or $C_1$- or $C_2$-haloalkyl,
or an environmentally compatible salt of the compound I, for example an alkali metal or alkaline earth metal salt or a manganese, copper, zinc, iron, ammonium, phosphonium, sulfonium or sulfoxonium salt.

**2.** A process for the preparation of a compound of the formula I as claimed in claim 1, which comprises reacting a corresponding salicylic acid derivative or (thio)salicylic acid derivative of the formula II

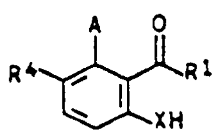

II

by a conventional method with a heterocycle of the formula III

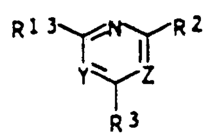

III

where $R^{13}$ is a nucleofugic leaving group, in the presence of an inorganic or organic base.

**3.** A process for the preparation of a compound of the formula I as claimed in claim 1, which comprises first converting the free salicylic acid or its sulfur analog of the formula I'

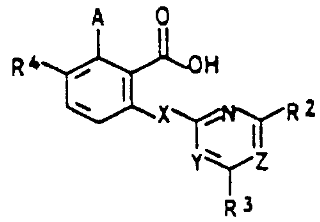

I'

in a conventional manner into the halide or another activated form of the carboxylic acid and then reacting this in the presence or absence of an inorganic or organic base with an unsubstituted or

substituted alcohol, an azole, an oxime or N-hydroxysuccinylimine.

**4.** A herbicide containing a compound of the formula I as claimed in claim 1.

**5.** A method for controlling undesirable plant growth, wherein the undesirable plants and/or their habitat are (is) treated with a herbicidally effective amount of a derivative I as claimed in claim 1.

**6.** The use of a salicylaldehyde derivative or salicylic acid derivative or sulfur analog thereof of the formula I as claimed in claim 1 for controlling undesirable plant growth.

**7.** An agent for influencing plant growth, containing a salicylaldehyde derivative or salicylic acid derivative of the formula I as claimed in claim 1.

**8.** A method for regulating plant growth, wherein a regulatory amount of a salicylaldehyde derivative or salicylic acid derivative of the formula Ia as claimed in claim 1 is allowed to act on the seeds, plants and/or their habitat.

**9.** A salicylic acid derivative of the formula II'

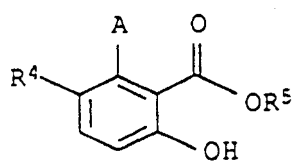

II'

where

$R^5$ is hydrogen, an alkali metal cation, one equivalent of an alkaline earth metal cation or an organic ammonium ion;

$C_1$-$C_{10}$-alkyl which may carry from one to five halogen atoms and/or one of the following radicals: $C_1$-$C_4$-alkoxy, $C_1$-$C_4$-alkylthio, phenyl or phenoxy, where the phenyl radicals may each carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;

$R^4$ is hydrogen, halogen, $C_1$-$C_4$-alkyl, cyano or $C_1$-$C_4$-haloalkyl;

A is a 5-membered heteroaromatic radical having from two to four nitrogen atoms or one or two nitrogen atoms and additionally one sulfur or oxygen atom in the ring, which may carry from one to three halogen atoms and/or from one to three of the following radicals: nitro, cyano, $C_1$-$C_4$-alkyl, $C_1$-$C_4$-alkylthio, $C_1$-$C_4$-haloalkyl or phenyl which is unsubstituted or substituted by from one to three halogen atoms and/or from one to three methyl groups;

thienyl which may carry from one to three halogen atoms and/or from one to three of the following radicals: $C_1$-$C_4$-alkyl, $C_1$- or $C_2$-haloalkyl or nitro;

a naphthyl, quinolyl, indazolyl or benzotriazolyl radical, each of which may carry from one to three halogen atoms and/or from one to three of the following radicals: $C_1$-$C_4$-alkyl or $C_1$- or $C_2$-haloalkyl,

**10.** A salicylaldehyde derivative or salicylic acid derivative of the formula I as claimed in claim 1, where $R^2$ is $OCH_3$, X is oxygen, Y is nitrogen, Z is a methine group and A, $R^1$, $R^3$ and $R^4$ have the meanings given in claim 1.

**11.** A salicylic acid derivative of the formula I as claimed in claim 1, where $R^1$ is OH, $R^2$ and $R^3$ are each $OCH_3$, $R^4$ is hydrogen, X is oxygen, Y is nitrogen, Z is a methine group and A is phenyl.

## Revendications

**1.** Dérivés d'aldéhyde et d'acide salicylique et leurs analogues à base de soufre de formule I

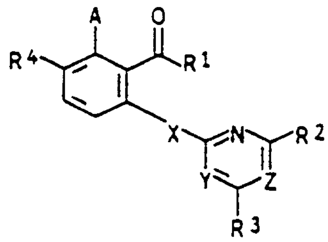

I

dans laquelle les substituants ont la signification suivante :

$R^1$, hydrogène ;
un groupe succinyliminooxy ;
un hétéroaromate à 5 chaînons, contenant un à trois atomes d'azote, qui peut porter un à quatre atomes d'halogène et/ou un à deux des restes suivants : alkyle en $C_1$-$C_4$, halogénalkyle en $C_1$-$C_4$, alcoxy en $C_1$-$C_4$, halogénalcoxy en $C_1$-$C_4$ et/ou alkyle en $C_1$-$C_4$-thio ;
un reste -$OR^5$ ou un reste ON = $CR^6R^7$, où
$R^5$ représente hydrogène, un cation de métal alcalin, l'équivalent d'un cation de métal alcilino terreux ou un ion ammonium
un groupe cycloalkyle en $C_3$-$C_{12}$ qui peut porter un à trois restes alkyle en $C_1$-$C_4$
un groupe alkyle en $C_1$-$C_{10}$ qui peut porter un à cinq atomes d'halogène et/ou un des restes suivants : alcoxy en $C_1$-$C_4$,alkyle en $C_1$-$C_4$-thio, cyano, alkyle en $C_1$-$C_8$-carbonyle, alcoxy en $C_1$-$C_8$-carbonyle, cycloalkyle en $C_3$-$C_{12}$, phényle, phénoxy ou phénylcarbonyle, les restes aromatiques à leur tour peuvent porter un à cinq atomes d'halogène et/ou un à trois des restes suivants : alkyle en $C_1$-$C_4$, halogénalkyle en $C_1$-$C_4$, alcoxy en $C_1$-$C_4$, halogénoalcoxy en $C_1$-$C_4$ et/ou alkylthio en $C_1$-$C_4$ ;
un groupe alkyle en $C_2$-$C_6$ qui porte en position 2 un des restes suivants : alcoxy en C1-C6 imino, alcényl en $C_3$-$C_6$ oxyimino, halogénalcényl en $C_3$-$C_6$ oxyimino ou benzyloxyimino ;
un groupe alcényle en $C_3$-$C_6$ ou alkinyle en $C_3$-$C_6$, ces groupes à leur tour pouvant porter un à cinq atomes d'halogène ;
phényle non substitué ou substitué une à trois fois par alkyle en $C_1$-$C_4$ ou alcoxy en $C_1$-$C_4$ ou substitué une à cinq fois par halogène et
$R^6$ et $R^7$ représentent
alkyle en $C_1$-$C_{20}$ qui peut porter un reste phényle, un groupe alcoxy en $C_1$-$C_4$ et/ou alkyle en $C_1$-$C_4$-thio, phényle ou ensemble une chaîne alkylène en $C_3$-$C_{12}$ qui peut porter un à trois groupes alkyle en $C_1$-$C_3$ .

$R^2$, $R^3$ alkyle en $C_1$-$C_4$, halogénalkyle en $C_1$-$C_4$, alcoxy en $C_1$-$C_4$, halogénalcoxy en $C_1$-$C_4$ et/ou alkyle en $C_1$-$C_4$-thio ;

X un atome d'oxygène ou de soufre

Y, Z un atome d'azote ou un groupe méthine =CH-

$R^4$, hydrogène, halogène, alkyle en $C_1$-$C_4$, cyano ou halogénalcoxy en $C_1$-$C_4$ ;

A un reste phényle substitué une à cinq fois, éventuellement substitué une à trois fois, dans le cas de l'halogène comme substituant

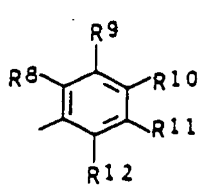

où
$R^8$ - $R^{12}$ représentent hydrogène, halogène, cyano, nitro, un groupe alcényle en $C_3$-$C_6$, alcényle en $C_3$-$C_6$-oxy, alcinyle en C3-C6-oxy ou alcinyle en $C_3$-$C_6$, ces groupes à leur tour pouvant porter un à cinq atomes d'halogène ;
Di-alkyle en $C_1$-$C_4$-amino, cycloalkyle en $C_3$-$C_8$ qui peut porter un à trois restes alkyle en $C_1$-$C_4$
alcoxy en $C_1$-$C_{10}$-carbonyle, alkyle en $C_1$-$C_4$-thio ;
un groupe phénoxy, le reste aromatique pouvant porter un à cinq atomes d'halogène et/ou un à trois des restes suivants : alkyle en $C_1$-$C_4$, halogénalkyle en $C_1$-$C_4$, halogénalcoxy en $C_1$-$C_4$, alcoxy en $C_1$-$C_4$, alkyle en $C_1$-$C_4$-thio ;
un groupe alkyle en $C_1$-$C_{10}$ ou alcoxy en $C_1$-$C_{10}$ qui peut porter un à cinq atomes d'halogène et/ou un des restes suivants : alcoxy en $C_1$-$C_4$, alkyle en $C_1$-$C_4$-thio, phényle ou phénoxy, les restes aromatiques à leur tour pouvant porter un à cinq atomes d'halogène et/ou un à trois des restes suivants : alkyle en $C_1$-$C_4$, halogénalkyle en $C_1$-$C_4$, halogénalcoxy en $C_1$-$C_4$, alkyle en $C_1$-$C_4$-thio ;
un hétéroaromate à 5 chaînons, avec deux à quatre atomes d'azote ou un à deux atomes d'azote ainsi additionnellement un atome de soufre ou d'oxygène dans le cycle, qui peut porter un à trois atomes d'halogène et/ou un à trois des restes suivants : nitro, cyano, alkyle en $C_1$-$C_4$, alkyle en $C_1$-$C_4$-thio, halogénalkyle en $C_1$-$C_4$, ou phényle qui n'est pas substitué ou est substitué par un à trois atomes d'halogène et/ou un à trois groupes méthyle ;
un reste thiényle qui peut porter un à trois atomes d'halogène et/ou un à trois des restes suivants : alkyle en $C_1$-$C_4$, halogénalkyle en $C_1$-$C_2$ ou nitro ;
un reste pyridyle, qui peut porter un à trois atomes d'halogène et/ou un à trois des restes suivants : alkyle en $C_1$-$C_4$, halogénalkyle en $C_1$-$C_2$ ou nitro ;
un reste naphtyle, quinoline, indazolyle ou benzotriazolyle qui peut porte chacun un à trois atomes d'halogène et/ou un à trois des restes suivants : alkyle en $C_1$-$C_4$, halogénalkyle en $C_1$-$C_2$ ainsi que des sels des composés I acceptables pour l'environnement, par exemple des sels de métaux alcalins de métaux alcalino-terreux, de manganèse, cuivre, zinc ou fer ainsi que des sels d'ammonium, phosphonium ou sulfonium.

**2.** Procédé de préparation de composés de formule I selon la revendication 1, caractérisé par le fait que l'on fait réagir, de manière connue en soi, en présence d'une base organique ou inorganique, un dérivé d'acide salicylique ou (thio)salicylique de formule II

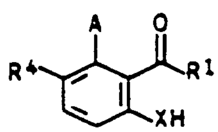

II

avec un hétérocycle de la formule III

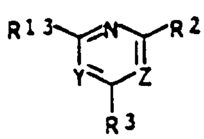

III

où $R^{13}$ est mis pour un groupe éliminable nucléofuge.

**3.** Procédé de préparation de composés de formule I selon la revendication 1, caractérisé par le fait que l'on transforme l'acide salicylique libre ou son analogue à base de soufre, de formule I'

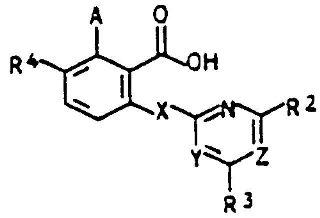

I'

tout d'abord, de manière connue en soi, en l'halogénure ou une autre forme activée de l'acide carboxylique et on fait réagir celui-ci, éventuellement en présence d'une base organique ou inorganique, avec un alcool éventuellement substitué, un azole, un oxime ou N-hydroxysuccinylimine.

**4.** Agent herbicide contenant un composé de formule I selon la revendication 1.

**5.** Procédé pour lutter contre la croissance des plantes indésirables, caractérisé par le fait que l'on traite les plantes indésirables et/ou leur biotope avec une quantité active au point de vue herbicide d'un dérivé I selon la revendication 1.

**6.** Utilisation de dérivés d'acide et d'aldéhyde salicylique et leurs analogues à base de soufre, de formule I selon la revendication 1 pour lutter contre la croissance des plantes indésirables.

**7.** Moyen pour influencer sur la croissance des plantes, contenant un dérivé d'acide ou d'aldéhyde salicylique de formule I selon la revendication 1.

**8.** Procédé de régularisation de la croissance des plantes, caractérisé par le fait que l'on fait agir une quantité active au point de vue régulation d'un dérivé d'acide ou aldéhyde salicylique de la formule générale I selon la revendication 1, sur les semences, les plantes et/ou leur biotope.

**9.** Dérivés d'acide salicylique de la formule générale II'

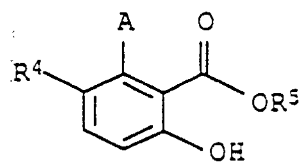

II'

dans laquelle les substituants ont la signification suivante :

$R^5$ hydrogène, un cation de métal alcalin, l'équivalent d'un cation de métal alcalino-terreux, un ion ammonium organique, un groupe alkyle en $C_1$-$C_{10}$ qui peut porter un à cinq atomes d'halogène et/ou un des restes suivants : alcoxy en $C_1$-$C_4$, alkyle en $C_1$-$C_4$-thio, phényle, phénoxy, les restes phényle pouvant porter un à cinq atomes d'halogène et/ou un à trois des restes suivants : alkyle en $C_1$-$C_4$, halogénalkyle en $C_1$-$C_4$, alcoxy en $C_1$-$C_4$, halogénalcoxy et/ou alkyle en $C_1$-$C_4$-thio ;

$R^4$ hygrogène, un atome d'halogène alkyle en $C_1$-$C_4$, cyano ou halogénoalkyle en $C_1$-$C_4$ ;

A un hétéroatome à 5 chaînons, avec deux à quatre atomes d'azote ou un à deux atomes d'azote ainsi qu'en plus un atome de soufre d'oxygène dans le cycle, qui peut porte un à trois atomes d'halogène et/ou un à trois des restes suivants : nitro, cyano, alkyle en $C_1$-$C_4$, alkyle en $C_1$-$C_4$-thio, halogénalkyle en $C_1$-$C_4$ ou phényle qui n'est pas substitué ou est substitué par un à trois atomes d'halogène et/ou un à trois groupes méthyle ;
un reste thiényle qui peut porter un à trois atomes d'halogène et/ou un à trois des restes suivants : alkyle en $C_1$-$C_4$, halogénoalkyle en $C_1$-$C_2$ ou nitro ;
un reste naphtyle, quinoline, indazolyle ou benzotriazolyle qui peut chacun porter un à trois atomes d'halogène et/ou un à trois des restes suivants : alkyle en $C_1$-$C_4$ ou halogénalkyle en $C_1$-$C_2$.

**10.** Dérivés d'acide et d'aldéhyde salicylique de formule I selon la revendication 1, dans laquelle $R^2$ représente $OCH_3$, X, oxygène, Y azote, Z le groupe méthine et A ainsi que les restes $R^1$, $R^3$ et $R^4$ ont la signification indiquée dans la revendication 1.

**11.** Dérivé d'acide salicylique de formule I selon la revendication 1, dans laquelle $R^1$ représente OH, $R^2$ et $R^3$, $OCH_3$, $R^4$, hydrogène, X, oxygène, Y, azote, Z, le groupe méthine et A, phényle.